(19) Europäisches Patentamt
European Patent Office
Office européen des brevets

(11) EP 1 749 821 A1

(12) EUROPEAN PATENT APPLICATION

(43) Date of publication:
**07.02.2007 Bulletin 2007/06**

(21) Application number: **05384026.0**

(22) Date of filing: **15.07.2005**

(51) Int Cl.:
***C07D 231/06*** *(2006.01)* ***A61K 31/4155*** *(2006.01)*
***A61P 25/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventors:
- **Torrens Jover, Antonio**
  **08221 Terrasa (Barcelona) (ES)**
- **Mas Prio, José**
  **08191-Rubi(Barcelona) (ES)**

(54) **Quaternary ammonium salts of substituted pyrazoline compounds, their preparation and use as medicaments**

(57) The present invention relates to quaternary ammonium salts of substituted pyrazoline compounds of formula I,

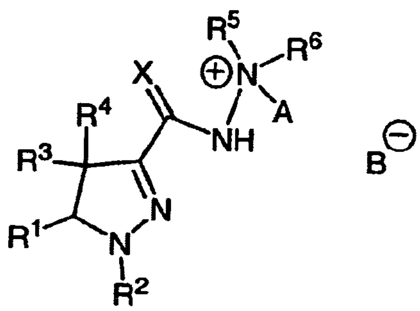

I

methods for their preparation, medicaments comprising theses compounds as well as their use for the preparation of a medicament for the treatment of humans and animals.

EP 1 749 821 A1

## Description

The present invention relates to quaternary ammonium salts of substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occurring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e. g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of cannabinoid receptors, more particularly for the modulation of cannabinoid 1 ($CB_1$) receptors.

Said object was achieved by providing the quaternary ammonium salts of substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e. g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to quaternary ammonium salts of substituted pyrazoline compounds of general formula I,

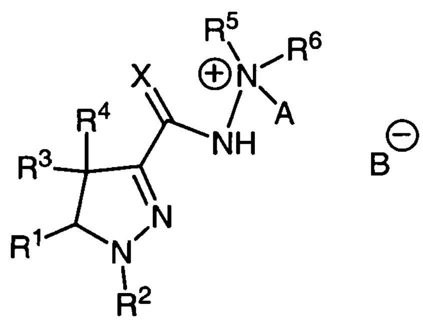

I

wherein

X is O or S;

A represents oxygen or an unsubstituted or at least mono-substituted alkyl radical;

$R^1$ and $R^2$, independently of one another, in each case represent an unsubstituted or at least mono-substituted aryl or heteroaryl radical, which may be condensed with an unsubstituted or at least mono-substituted mono- or polycyclic ring system;

or a saturated or unsaturated, unsubstituted or at least mono-substituted, optionally at least one heteroatom as a ring

member containing cycloaliphatic radical, which may be condensed with an unsubstituted or at least mono-substituted mono- or polycyclic ring system and/or may be bridged by at least one unsubstituted or at least mono-substituted alkylene group;

$R^3$ and $R^4$, independently of one another, in each case represent H; F; Cl; Br; I; -CN; -$NO_2$; -NC; -OH; -$NH_2$; -SH; -C(=O)-H; a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;

a -O-$R^7$ moiety; a -S-$R^8$ moiety; a -NH-$R^9$ moiety or a -$NR^{10}R^{11}$ moiety;

$R^5$ and $R^6$, independently of one another, in each case represent a hydrogen atom;

a saturated or unsaturated, unsubstituted or at least mono-substituted aliphatic radical;

or

$R^5$ and $R^6$ together with the bridging nitrogen form an unsubstituted or at least mono-substituted, saturated or unsaturated heterocyclic ring which may contain at least one further heteroatom as a ring member and/or which may be condensed with one or two optionally at least mono-substituted mono- or polycyclic ring systems;

$R^7$, $R^8$, $R^9$, $R^{10}$ and $R^{11}$, independently of one another, in each case represent a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, unsubstituted or at least mono-substituted benzoate or naphthoate, pyruvate, ascorbate, glycolate, nicotinate, phenylacetate,

$$R^{12}\text{-}SO_3^{\ominus} \quad \text{and} \quad R^{13}\text{-NH-}SO_3^{\ominus};$$

and

$R^{12}$ and $R^{13}$, independently of one another, in each case, represent a saturated or unsaturated, unsubstituted or at least mono-substituted aliphatic radical;

an unsubstituted or at least mono-substituted aryl or heteroaryl radical which may be bonded via an unsubstituted or at least mono-substituted alkylene group

or a saturated or unsaturated, unsubstituted or at least mono-substituted, optionally at least one heteroatom as a ring member containing cycloaliphatic radical, which may be bridged by at least one unsubstituted or at least mono-substituted alkylene group and/or may be bonded via an unsubstituted or at least mono-substituted alkylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

Most particularly preferred is a medicament comprising at least one substituted pyrazoline compound selected from the group consisting of:

Preferably the N-oxides of the following compounds are disclaimed

5-(4-Chloro-phenyl)-1-(2,4-dichloro-phenyl)-4,5-dihydro-1H-pyrazole-3-carboxylic acid-piperidin-1-yl-amide,

5-(4-Chloro-phenyl)-1-(2,4-dichloro-phenyl)-4,5-dihydro-1H-pyrazole-3-carboxylic acid-[1,2,4]-triazole-4-yl-amide,

and

5-(4-Chloro-phenyl)-1-(2,4-dichloro-phenyl)-4,5-dihydro-1H-pyrazole-3-carboxylic acid-(4-methyl-piperazin-1-yl)-amide.

If one or more of the residues $R^1$ to $R^{13}$ and B represents or comprises an aryl, heteroaryl, benzoate or naphthoate radical, which may be substituted, unless defined otherwise, preferably said aryl, heteroaryl, benzoate or naphthoate radical may optionally be substituted with 1, 2, 3, 4 or 5 substituent(s) independently selected from the group consisting of $C_{1-6}$-perfluoralkyl, -$C_{1-6}$-alkyl substituted with one or more methoxy and/or ethoxy groups, -$C_{1-6}$-alkyl, -$C_{1-6}$-alkyl substituted with one or more hydroxy groups, -$C_{1-6}$-alkyl substituted with one or more chlorine atoms, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I, -CN, -$OCF_3$, - O-$C_2F_5$, -O-$C_3F_7$, -O-$C_4F_9$, -$SCF_3$, -$SCF_3$, -$SCF_2H$, -$SCFH_2$, -OH, -SH, -$SO_3H$, -NH-C(=O)-$C_{1-6}$-alkyl, -N($C_{1-6}$-alkyl)-C(=O)-$C_{1-6}$-alkyl, -$NO_2$, -CHO, -C(=O)-$C_{1-6}$-alkyl, - C (=O)-$C_{1-6}$-perfluoroalkyl, -C(=S)-NH-$C_{1-6}$-alkyl, -$CF_2H$, -$CFH_2$, -C(=O)-$NR^AR^B$, - C(=O)-NH-$NR^CR^D$, -S(=O)-$C_{1-6}$-alkyl, -S(=O)$_2$-$C_{1-6}$-alkyl, -S(=O)$_2$-phenyl, -($C_{1-5}$-alkylene)-S-$C_{1-6}$-alkyl, -($C_{1-5}$-alkylene)-S(=O)-$C_{1-6}$-alkyl, -($C_{1-5}$-alkylene)-S (=O)$_2$-$C_{1-6}$-alkyl, -$NR^ER^F$, -($C_{1-5}$-alkylene)-$NR^ER^F$, -S(=O)-$NH_2$, -S(=O)$_2$-NH-$C_{1-6}$-alkyl, -S(=O)$_2$-NH-phenyl, -NH-S (=O)$_2$-$C_{1-6}$-alkyl, cyclopropyl, cyclobutyl, cyclopentyl, cyclohexyl, pyrrolidinyl, piperidinyl, phenyl, thiophenyl, phenoxy and benzyl;

whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and whereby $R^A$, $R^B$, $R^E$ and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, -$C_{1-6}$-alkyl, -C(=O)-O-$C_{1-6}$-alkyl, $C_{3-8}$-cycloalkyl, -($C_{1-5}$-alkylene)-$C_{3-8}$-cycloalkyl, -($C_{1-6}$-alkylene)-O-$C_{1-6}$-alkyl or -$C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -$C_{1-6}$-alkyl, -C(=O)-$C_{1-6}$-alkyl, -C(=O)-O-$C_{1-6}$-alkyl, -C(=O)-NH-$C_{1-6}$-alkyl, -C(=S)-NH-$C_{1-6}$-alkyl, oxo (=O), -$C_{1-6}$-alkyl substituted with one or more hydroxy groups, -($C_{1-6}$-alkylene)-O-$C_{1-6}$-alkyl and —C(=O)-$NH_2$.

More preferably said aryl and heteroaryl radicals may optionally be substituted with 1, 2, 3, 4 or 5 substituent (s) independently selected from the group consisting of -$CF_3$, - $C_2F_5$, -$C_3F_7$, -$C_4F_9$, -$CH_2Cl$, -$CHCl_2$, -$C_2H_4Cl$, methyl, ethyl, n-propyl, isopropyl, n-butyl, isobutyl, 2-butyl, tert-butyl, n-pentyl, 2-pentyl, n-hexyl, -$CH_2$-OH, -$CH_2$-$CH_2$-OH, -$CH_2$-$CH_2$-$CH_2$-OH, -O-$CH_2$-O-$CH_3$, -O-$CH_2$-$CH_2$-O-$CH_3$, -O-$CH_2$-O-$C_2H_5$, - C($OCH_3$)($C_2H_5$)$_2$, -C($OCH_3$)($CH_3$)$_2$, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, -C(=O)-OH, -C(=O)-O-$CH_3$, -C(=O)-O-$C_2H_5$, - C (=O)-O-$C_3H_7$, -C(=O)-O-C($CH_3$)$_3$, -O-C(=O)-$CH_3$, -O-C(=O)-$C_2H_5$, -O-C(=O)-CH($CH_3$)$_2$, -O-C(=O)-$CH_2$-$CH_2$-$CH_3$, -O-C(=O)-C($CH_3$)$_3$, F, Cl, Br, I, -CN, -$OCF_3$, -0-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$CH_3$, -NH-C(=O)-$C_2H_5$, -NH-C(=O)-C($CH_3$)$_3$, -$NO_2$, -CHO, -C(=O)-$CH_3$, -C(=O)-$C_2H_5$, - C(=O)-C($CH_3$)$_3$, -C (=O)-$CF_3$, -C(=O)-$C_2F_5$, -C(=O)-$C_3F_7$, -C(=S)-NH-$CH_3$, -C(=S)-NH-$C_2H_5$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -C(=O)-NH-NH-$CH_3$, -C(=O)-NH-NH-$C_2H_5$, -C(=O)-NH-$NH_2$, -C(=O)-NH-N($CH_3$)$_2$, -S(=O)-$CH_3$, -S(=O)-$C_2H_5$, -S(=O)-$C_3H_7$, - S(=O)$_2$-$CH_3$, -S(=O)$_2$-$C_2H_5$, -S (=O)$_2$-$C_3H_7$, -S(=O)$_2$-phenyl, -$NH_2$, -NH-$CH_3$, -NH-$C_2H_5$, -N($CH_3$)$_2$, -N($C_2H_5$)$_2$, -$CH_2$-N($CH_3$)$_2$, -($CH_2$)-morpholinyl, -($CH_2$)-piperidinyl, -($CH_2$)-piperazinyl, -($CH_2$)-N($C_2H_5$)$_2$, -$CH_2$-N($C_3H_7$)$_2$, -$CH_2$-N($C_4H_9$)$_2$, -$CH_2$-N($CH_3$)($C_2H_5$), - S (=O)-$NH_2$, -S(=O)$_2$-NH-$CH_3$, -S(=O)$_2$-NH-phenyl, -NH-S(=O)$_2$-$CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituent(s) independently selected from the group consisting of F, Cl, Br, methyl, ethyl and n-propyl.

Preferred aryl radicals which are optionally at least mono-substituted are phenyl and naphthyl (1- and 2-naphthyl).

Preferably the heteroatoms which are present as ring members in the heteroaryl radical may, unless defined otherwise, independently be selected from the group consisting of nitrogen, oxygen and sulfur. More preferably a heteroaryl radical is 5- to 14-membered and may comprise 1, 2, 3 or 4 heteroatoms independently selected from the group

consisting of nitrogen, oxygen and sulfur.

Preferred heteroaryl radicals which are unsubstituted or at least mono-substituted are pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl, benzimidazolyl and quinazolinyl.

Preferred aryl and heteroaryl radicals which are condensed with a mono- or polycyclic ring system are [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4J-tetrahydroquinazolinyl and [3,4]-dihydro-2H-benzo[1,4]oxazinyl.

If one or more of the residues $R^1$ to $R^{13}$ and B represents or comprises a saturated or unsaturated, optionally at least one heteroatom as a ring member containing cycloaliphatic radical, preferably a $C_{1-18}$ cycloaliphatic radical, which may be substituted, unless defined otherwise, preferably said cycloaliphatic radical may optionally be substituted with 1, 2, 3, 4 or 5 substituent(s) independently selected from the group consisting of oxo (=O), thioxo (=S), $C_{1-6}$-perfluoralkyl, -$C_{1-6}$-alkyl, -$C_{1-6}$-alkyl substituted with one or more hydroxy groups, -$C_{1-6}$-alkyl substituted with one or more chlorine atoms, -$C_{1-6}$-alkyl substituted with one or more methoxy and/or ethoxy groups, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I, -CN, -$OCF_3$, -O-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$C_{1-6}$-alkyl, -N($C_{1-6}$-alkyl)-C(=O)-$C_{1-6}$-alkyl, -$NO_2$, -CHO, -C(=O)-$C_{1-6}$-alkyl, -C(=O)-$C_{1-6}$-perfluoroalkyl, -C(=S)-NH-$C_{1-6}$-alkyl, -$CF_2H$, -$CFH_2$, -C(=O)-$NR^AR^B$, -C(=O)-NH-$NR^CR^D$, -S(=O)-$C_{1-6}$-alkyl, -$S(=O)_2$-$C_{1-6}$-alkyl, -$S(=O)_2$-phenyl, - ($C_{1-5}$-alkylene)-S-$C_{1-6}$-alkyl, -($C_{1-5}$-alkylene)-S(=O)-$C_{1-6}$-alkyl, -($C_{1-5}$-alkylene)-$S(=O)_2$-$C_{1-6}$-alkyl, -$NR^ER^F$, -($C_{1-5}$-alkylene)-$NR^ER^F$, -S(=O)-$NH_2$, -$S(=O)_2$-NH-$C_{1-6}$-alkyl, - $S(=O)_2$-NH-phenyl, -NH-$S(=O)_2$-$C_{1-6}$-alkyl, cyclopropyl, cyclobutyl, cyclopentyl, cyclohexyl, pyrrolidinyl, piperidinyl, phenyl, thiophenyl, phenoxy and benzyl;

whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and whereby $R^A$, $R^B$, $R^E$ and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, -$C_{1-6}$-alkyl, -C(=O)-O-$C_{1-6}$-alkyl, $C_{3-8}$-cycloalkyl, -($C_{1-5}$-alkylene)-$C_{3-8}$-cycloalkyl, -($C_{1-6}$-alkylene)-O-$C_{1-6}$-alkyl or -$C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -$C_{1-6}$-alkyl, -C(=O)-$C_{1-6}$-alkyl, -C(=O)-O-$C_{1-6}$-alkyl, -C(=O)-NH-$C_{1-6}$-alkyl, -C(=S)-NH-$C_{1-6}$-alkyl, oxo (=O), -$C_{1-6}$-alkyl substituted with one or more hydroxy groups, -($C_{1-6}$-alkylene)-O-$C_{1-6}$-alkyl and —C(=O)-$NH_2$.

More preferably said cycloaliphatic radicals may optionally be substituted with 1, 2, 3, 4 or 5 substituent(s) independently selected from the group consisting of oxo (=O), thioxo (=S), —$CF_3$, -$C_2F_5$, -$C_3F_7$, -$C_4F_9$, -$CH_2Cl$, -$CHCl_2$, -$C_2H_4Cl$, methyl, ethyl, n-propyl, isopropyl, n-butyl, isobutyl, 2-butyl, tert-butyl, n-pentyl, 2-pentyl, n-hexyl, - $CH_2$-OH, -$CH_2$-$CH_2$-OH, -$CH_2$-$CH_2$-$CH_2$-OH, -O-$CH_2$-O-$CH_3$, -O-$CH_2$-$CH_2$-O-$CH_3$, -0-$CH_2$-O-$C_2H_5$, -C($OCH_3$)($C_2H_5$)$_2$, -C($OCH_3$)($CH_3$)$_2$, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, -C(=O)-OH, -C(=O)-O-$CH_3$, -C(=O)-O-$C_2H_5$, -C(=O)-O-$C_3H_7$, -C(=O)-O-C($CH_3$)$_3$, -O-C(=O)-$CH_3$, -O-C(=O)-$C_2H_5$, -0-C(=O)-CH($CH_3$)$_2$, -O-C(=O)-$CH_2$-$CH_2$-$CH_3$, -O-C(=O)-C($CH_3$)$_3$, F, Cl, Br, I, -CN, - $OCF_3$, -O-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$CH_3$, -NH-C(=O)-$C_2H_5$, -NH-C(=O)-C($CH_3$)$_3$, -$NO_2$, -CHO, -C(=O)-$CH_3$, -C(=O)-$C_2H_5$, -C(=O)-C($CH_3$)$_3$, -C(=O)-$CF_3$, -C(=O)-$C_2F_5$, -C(=O)-$C_3F_7$, -C(=S)-NH-$CH_3$, - C(=S)-NH-$C_2H_5$, -$CF_2H$, -$CFH_2$, -C(=O)-$NH_2$, -C(=O)-NH-$CH_3$, -C(=O)-NH-$C_2H_5$, - C(=O)-NH-$C_3H_7$, -C(=O)-N($CH_3$)$_2$, -C(=O)-N($C_2H_5$)$_2$, -C(=O)-NH-NH-$CH_3$, -C(=O)-NH-NH-$C_2H_5$, -C(=O)-NH-$NH_2$, -C(=O)-NH-N($CH_3$)$_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$S(=O)_2$-phenyl, -$NH_2$, -NH-$CH_3$, - NH-$C_2H_5$, -N($CH_3$)$_2$, -N($C_2H_5$)$_2$, -$CH_2$-N($CH_3$)$_2$, -($CH_2$)-morpholinyl, -($CH_2$)-piperidinyl, -($CH_2$)-piperazinyl, -($CH_2$)-N($C_2H_5$)$_2$, -$CH_2$-N($C_3H_7$)$_2$, -$CH_2$-N($C_4H_9$)$_2$, -$CH_2$-N($CH_3$)($C_2H_5$), -S(=O)-$NH_2$, -$S(=O)_2$-NH-$CH_3$, -$S(=O)_2$-NH-phenyl, -NH-$S(=O)_2$-$CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^1$ to $R^{13}$ and B represents or comprises a cycloaliphatic radical, preferably a $C_{3-16}$ cycloaliphatic radical, which contains one or more heteroatoms as ring members, unless defined otherwise, each of these heteroatoms may preferably be selected from the group consisting of nitrogen, oxygen and sulfur. More preferably a cycloaliphatic group may optionally contain 1, 2, 3 or 4 heteroatom(s) independently selected from the group consisting

of N, O and S as ring members.

Suitable saturated or unsaturated, optionally at least one heteroatom as ring member containing cycloaliphatic radicals may preferably be selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals which are condensed with an unsubstituted or at least mono-substituted mono- or polycyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl and (1,2,3,4)-tetrahydroquinoxazlinyl.

Suitable saturated or unsaturated, optionally at least one heteroatom as ring member containing cycloaliphatic radicals which are bridged by at least one unsubstituted or at least mono-substituted alkylene group may preferably be selected from the group consisting of adamantyl, bicyclo[2.2.1]heptyl, bicyclo[3.1.1]heptyl, norbornenyl and 8-aza-bicyclo [3.2.1]octyl.

A mono- or polycyclic ring system according to the present invention — if not defined otherwise - means a mono- or polycyclic hydrocarbon ring system, preferably a mono- or bicyclic ring system, that may be saturated, unsaturated or aromatic. Each of its different rings may show a different degree of saturation, i.e. they may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of nitrogen, oxygen and sulfur. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

If one or more of the residues $R^1$ to $R^{11}$ comprises a mono- or polycyclic ring system, which may be substituted, unless defined otherwise, preferably said mono- or polycyclic ring system may optionally be substituted with 1, 2, 3, 4 or 5 substituent(s) independently selected from the group consisting of oxo (=O), thioxo (=S), $C_{1-6}$-perfluoralkyl, -$C_{1-6}$-alkyl, -$C_{1-6}$-alkyl substituted with one or more hydroxy groups, -$C_{1-6}$-alkyl substituted with one or more chlorine atoms, -$C_{1-6}$-alkyl substituted with one or more methoxy and/or ethoxy groups, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I, -CN, -$OCF_3$, -O-$C_2F_5$, -O-$C_3F_7$, -O-$C_4F_9$, -$SCF_3$, -$SCF_2H$, -$SCFH_2$, -OH, -SH, -$SO_3H$, -NH-C(=O)-$C_{1-6}$-alkyl, -N($C_{1-6}$-alkyl)-C(=O)-$C_{1-6}$-alkyl, -$NO_2$, -CHO, -C(=O)-$C_{1-6}$-alkyl, -C(=O)-$C_{1-6}$-perfluoroalkyl, -C(=S)-NH-$C_{1-6}$-alkyl, -$CF_2H$, -$CFH_2$, -C(=O)-$NR^AR^B$, -C(=O)-NH-$NR^CR^D$, -S(=O)-$C_{1-6}$-alkyl, -$S(=O)_2$-$C_{1-6}$-alkyl, -$S(=O)_2$-phenyl, -($C_{1-5}$-alkylene)-S-$C_{1-6}$-alkyl, -($C_{1-5}$-alkylene)-S(=O)-$C_{1-6}$-alkyl, - ($C_{1-5}$-alkylene)-$S(=O)_2$-$C_{1-6}$-alkyl, -$NR^ER^F$, -($C_{1-5}$-alkylene)-$NR^ER^F$, -S(=O)-$NH_2$, - $S(=O)_2$-NH-$C_{1-6}$-alkyl, -$S(=O)_2$-NH-phenyl, -NH-$S(=O)_2$-$C_{1-6}$-alkyl, cyclopropyl, cyclobutyl, cyclopentyl, cyclohexyl, pyrrolidinyl, piperidinyl, phenyl, thiophenyl, phenoxy and benzyl;

whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and

whereby $R^A$, $R^B$, $R^E$ and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, -$C_{1-6}$-alkyl, -C(=O)-O-$C_{1-6}$-alkyl, $C_{3-8}$-cycloalkyl, -($C_{1-5}$-alkylene)-$C_{3-8}$-cydoalkyl, -($C_{1-6}$-alkylene)-O-$C_{1-6}$-alkyl or -$C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -$C_{1-6}$-alkyl, -C(=O)-$C_{1-6}$-alkyl, -C(=O)-O-$C_{1-6}$-alkyl, -C(=O)-NH-$C_{1-6}$-alkyl, -C(=S)-NH-$C_{1-6}$-alkyl, oxo (=O), -$C_{1-6}$-alkyl substituted with one or more hydroxy groups, -($C_{1-6}$-alkylene)-O-$C_{1-6}$-alkyl and -C(=O)-$NH_2$.

More preferably said mono- or polycyclic ring system may optionally be substituted with 1, 2, 3, 4 or 5 substituent(s) independently selected from the group consisting of oxo (=O), thioxo (=S), —$CF_3$, -$C_2F_5$, -$C_3F_7$, -$C_4F_9$, -$CH_2Cl$, -$CHCl_2$, -$C_2H_4Cl$, methyl, ethyl, n-propyl, isopropyl, n-butyl, isobutyl, 2-butyl, tert-butyl, n-pentyl, 2-pentyl, n-hexyl, -$CH_2$-OH, -$CH_2$-$CH_2$-OH, -$CH_2$-$CH_2$-$CH_2$-OH, -O-$CH_2$-O-$CH_3$, -O-$CH_2$-$CH_2$-O-$CH_3$, -O-$CH_2$-O-$C_2H_5$, -C($OCH_3$)($C_2H_5$)$_2$, -C($OCH_3$)($CH_3$)$_2$, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, -C(=O)-OH, -C(=O)-O-$CH_3$, -C(=O)-O-$C_2H_5$, -C(=O)-O-$C_3H_7$, -C(=O)-O-C($CH_3$)$_3$, -O-C(=O)-$CH_3$, -0-C(=O)-$C_2H_5$, -O-C(=O)-CH($CH_3$)$_2$, -O-C(=O)-$CH_2$-$CH_2$-$CH_3$, -O-C(=O)-C($CH_3$)$_3$, F, Cl, Br, I, -CN, -$OCF_3$, -O-$C_2F_5$, -O-$C_3F_7$, -O-$C_4F_9$, -$SCF_3$, -$SCF_2H$, -$SCFH_2$, -OH, -SH, - $SO_3H$, -NH-C(=O)-$CH_3$, -NH-C(=O)-$C_2H_5$, -NH-C(=O)-C($CH_3$)$_3$, -$NO_2$, -CHO, -C(=O)-$CH_3$, -C(=O)-$C_2H_5$, -C(=O)-C($CH_3$)$_3$, -C(=O)-$CF_3$, -C(=O)-$C_2F_5$, -C(=O)-$C_3F_7$, -C(=S)-NH-$CH_3$, -C(=S)-NH-$C_2H_5$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -C(=O)-NH-NH-$CH_3$, - C(=O)-NH-NH-$C_2H_5$, -C(=O)-NH-$NH_2$, -C(=O)-NH-N($CH_3$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)-$C_3H_7$, -S(=O)$_2$-phenyl, -$NH_2$, -NH-$CH_3$, -NH-$C_2H_5$, -N($CH_3$)$_2$, -N($C_2H_5$)$_2$, -$CH_2$-N($CH_3$)$_2$, -($CH_2$)-morpholinyl, -($CH_2$)-piperidinyl, -($CH_2$)-piperazinyl, -($CH_2$)-N($C_2H_5$)$_2$, -$CH_2$-N($C_3H_7$)$_2$, -$CH_2$-N($C_4H_9$)$_2$, -$CH_2$-N($CH_3$)($C_2H_5$), -S(=O)-$NH_2$, -S(=O)$_2$-NH-$CH_3$, -S(=O)$_2$-NH-phenyl, -NH-S(=O)$_2$-$CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^3$ to $R^{13}$, A and B represent or comprise a saturated or unsaturated, unsubstituted or at least mono-substituted aliphatic radical, preferably a $C_{1-16}$ aliphatic radical, said aliphatic radical may be linear or branched.

Preferably said aliphatic radicals, unless defined otherwise, may optionally be substituted with 1, 2, 3, 4, 5, 6, 7, 8 or 9 substituents independently selected from the group consisting of —OH, F, Cl, Br, I, -O-$C_{1-6}$-alkyl, -$OCF_3$, -O-$C_2F_5$, -O-$C_3F_7$, -O-$C_4F_9$, -$CF_3$, -$C_2F_5$, -$C_3F_7$, -$C_4F_9$, -$NH_2$, -NH-$C_{1-6}$-alkyl, -N($C_{1-6}$-alkyl)$_2$, -C(=O)-OH, -C(=O)-O-$C_{1-6}$-alkyl, -C(=O)-$NH_2$, -C(=O)-NH-$C_{1-6}$-alkyl, -C(=O)-N($C_{1-6}$-alkyl)$_2$, -CN, -$NO_2$, - S(=O)-$NH_2$, -CHO, -C(=O)-$C_{1-6}$-alkyl, -S(=O)-$C_{1-6}$-alkyl, -S(=O)$_2$-$C_{1-6}$-alkyl, -NH-S(=O)-$C_{1-6}$-alkyl, -NH-C(=O)-O-$C_{1-6}$-alkyl and -NH-C(=O)-$C_{1-6}$-alkyl.

More preferably said aliphatic radicals may optionally be substituted with 1, 2, 3, 4, 5, 6, 7, 8 or 9 substituents independently selected from the group consisting of -OH, F, Cl, Br, I, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, - O-C($CH_3$)$_3$, -$NH_2$, -NH-$CH_3$, -NH-$C_2H_5$, -N($CH_3$)$_2$,-N($C_2H_5$)$_2$, -CN, -$NO_2$, -NH-C(=O)-$CH_3$, -NH-C(=O)-$C_2H_5$, -NH-C(=O)-C($CH_3$)$_3$, -NH-C(=O)-O-$CH_3$, -NH-C(=O)-O-$C_2H_5$, - NH-C(=O)-O-C($CH_3$)$_3$, -C(=O)-NH-$CH_3$, -C(=O)-NH-$C_2H_5$, -C(=O)-NH-C($CH_3$)$_3$, - C(=O)-N($CH_3$)$_2$, -C(=O)-N($C_2H_5$)$_2$, -C(=O)-OH, -C(=O)-O-$CH_3$, -C(=O)-O-$C_2H_5$, - C(=O)-O-C($CH_3$)$_3$, -C(=O)-$CH_3$, -C(=O)-$C_2H_5$ and -C(=O)-C($CH_3$)$_3$.

Suitable alkyl radicals, preferably $C_{1-16}$ alkyl radicals, are selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl, 2-(6-methyl)-octyl, n-nonyl, n-decyl, n-undecyl, n-dodecyl, n-tridecyl, n-tetradecyl, n-pentadecycl and n-hexadecyl.

Suitable at least mono-substituted alkyl radicals are selected from the group consisting of -$CF_3$, -$CH_2F$, -$CF_2H$, -$CH_2$-O-$CH_3$, -$C_2F_5$, -$CH_2$-$CH_2$-F, -$CH_2$-CN, -$CH_2$-OH, -$CH_2$-$CH_2$-CN, -$CH_2$-$CH_2$-OH, -$CH_2$-$CH_2$-$OCH_3$, -$CH_2$-$CH_2$-$CH_2$-CN, -$CH_2$-$CH_2$-$CH_2$-OH, -$CH_2$-$CH_2$-$CH_2$-O-$CH_3$, -$CH_2$-$CH_2$-$CH_2$-$CH_2$-O-$CH_3$, -$CH_2$-$NH_2$, -$CH_2$-N($CH_3$)$_2$, -$CH_2$-N($C_2H_5$)$_2$, -$CH_2$-CH-$NH_2$, -$CH_2$-$CH_2$-N($CH_3$)$_2$, -$CH_2$-$CH_2$-N($C_2H_5$)$_2$, - $CH_2$-$CH_2$-$CH_2$-$NH_2$, -$CH_2$-$CH_2$-$CH_2$-N($CH_3$)$_2$ and -$CH_2$-$CH_2$-$CH_2$-N($C_2H_5$)$_2$.

An alkenyl radical according to the present invention comprises at least one carbon-carbon double bond. Suitable alkenyl radicals, preferably $C_{2-16}$ alkenyl radicals, are selected from the group consisting of vinyl, n-propenyl, n-butenyl, n-pentenyl, n-hexenyl, n-heptenyl, n-octenyl, n-nonenyl, n-decenyl, n-undecenyl, n-dodecenyl, n-tridecenyl, n-tetradecenyl, n-pentadecenyl and n-hexadecenyl.

An alkinyl radical comprises at least one carbon-carbon triple bond. Suitable alkinyl radicals, preferably $C_{2-16}$ alkinyl radicals, are selected from the group consisting of ethinyl, propinyl, n-butinyl, n-pentinyl, n-hexinyl, n-octinyl, n-noninyl, n-decinyl, n-undecinyl, n-dodecinyl, n-tridecinyl, n-tetradecinyl, n-pentadecinyl and n-hexadecinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-$C_{1-6}$-alkyl, -S-$C_{1-6}$-alkyl, -F, Cl, Br, I, -CN, -$CF_3$, -$OCF_3$, -$SCF_3$,- OH, -SH, -$SO_3H$, -$NH_2$, -NH($C_{1-6}$-alkyl), -N($C_{1-6}$-alkyl)$_2$ and phenyl. More preferably said substituent(s) may be selected from the group consisting of -F, Cl, Br, I, -CN, - $CF_3$, -$OCF_3$, -$SCF_3$, -OH, -SH, -$SO_3H$, -$NH_2$, -NH-$CH_3$, -N($CH_3$)$_2$, -O-$CH_3$ and -O-$C_2H_5$. An alkenylene group comprises at least one carbon-carbon double bond, an alkinylene group comprises at least one carbon-carbon triple bond.

Suitable alkylene groups, preferably $C_{1-5}$-alkylene groups, include -($CH_2$)-, -CH($CH_3$)-, -CH(phenyl), -($CH_2$)$_2$-, -($CH_2$)$_3$-,-($CH_2$)$_4$-,-($CH_2$)$_5$ and -($CH_2$)$_6$-, suitable alkenylene groups, preferably $C_{2-5}$-alkenylene groups, include

-CH=CH-, $-CH_2-CH=CH-$ and - $CH=CH-CH_2-$ and suitable alkinylene groups, preferably $C_{2-5}$-alkinylene groups, include -C≡C-, $-CH_2-C≡C-$ and $-C≡C-CH_2-$.

Preferred are quaternary ammonium salts of substituted pyrazoline compounds of general formula I given above, wherein

X is O or S;

A represents a monovalent oxygen anion ($O^-$) or an unsubstituted or at least mono-substituted $C_{1-10}$ alkyl radical;

$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

$R^3$ and $R^4$, independently of one another, in each case represent H; F; Cl; Br; I; -CN; $-NO_2$; -NC; -OH; $-NH_2$; -SH; -C(=O)-H; an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group; a —O-$R^7$ moiety; a —S-$R^8$ moiety; a —NH-$R^9$ moiety or a —$NR^{10}R^{11}$ moiety;

$R^5$ and $R^6$, independently of one another, in each case represent a hydrogen atom;

an unsubstituted or at least mono-substituted $C_{1-16}$ alkyl radical, $C_{2-16}$ alkenyl radical or $C_{2-16}$ alkinyl radical;

or

$R^5$ and $R^6$ together with the bridging nitrogen form an unsubstituted or at least mono-substituted, saturated or unsaturated 4- to 10-membered heterocyclic ring which may optionally contain 1, 2 or 3 additional heteroatom(s) selected from the group consisting of sulfur, nitrogen and oxygen as ring member(s) and/or which may be condensed with one or two optionally at least mono-substituted mono- or polycyclic ring systems;

$R^7$, $R^8$, $R^9$, $R^{10}$ and $R^{11}$, independently of one another, in each case represent

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted

or at least mono-substituted $C_{1-5}$ alkylene group, $C_{2-5}$ alkenylene group or $C_{2-5}$ alkinylene group;
and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, unsubstituted or at least mono-substituted benzoate or naphthoate, pyruvate, ascorbate, glycolate, nicotinate, phenylacetate,

$$R^{12}\text{-}SO_3^{\ominus} \quad \text{and} \quad R^{13}\text{-}NH\text{-}SO_3^{\ominus};$$

and
$R^{12}$ and $R^{13}$, independently of one another, in each case, represent
an unsubstituted or at least mono-substituted $C_{1-16}$ alkyl radical, $C_{2-16}$ alkenyl radical or $C_{2-16}$ alkinyl radical;
an unsubstituted or at least mono-substituted 6- or 10-membered aryl radical, which may be bonded via an unsubstituted or at least mono-substituted $C_{1-5}$ alkylene group; an unsubstituted or at least mono-substituted 5- to 14-membered heteroaryl radical, which may be bonded via an unsubstituted or at least mono-substituted $C_{1-5}$ alkylene group, $C_{2-5}$ alkenylene group or $C_{2-5}$ alkinylene group;
an unsubstituted or at least mono-substituted $C_{3-16}$ cycloalkyl radical or $C_{4-16}$ cycloalkenyl radical, which in each case may be bonded via an unsubstituted or at least mono-substituted $C_{1-5}$ alkylene group and/or may be bridged by at least one unsubstituted or at least mono-substituted $C_{1-5}$ alkylene group;
or an unsubstituted or at least mono-substituted $C_{4-16}$ heterocycloalkyl radical or $C_{5-16}$ heterocycloalkenyl radical, which in each case may be bonded via an unsubstituted or at least mono-substituted $C_{1-5}$ alkylene group and/or may be bridged by at least one unsubstituted or at least mono-substituted $C_{1-5}$ alkylene group;
whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

Preference is also given to quaternary ammonium salts of substituted pyrazoline compounds of general formula I, wherein A represents a monovalent oxygen anion ($O^-$) or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $- O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $-NO_2$;
and $R^1$ to $R^{13}$, X and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Also preferred are quaternary ammonium salts of substituted pyrazoline compounds of general formula I given above, wherein $R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of $-CF_3$, $-CH_2-Cl$, methyl, ethyl, n-propyl, isopropyl, n-butyl, isobutyl, 2-butyl, tert-butyl, n-pentyl, 2-pentyl, n-hexyl, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $- S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, -C(=O)-NH-

$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, - N($CH_3$)$_2$, -N($C_2H_5$)$_2$, phenoxy and thiophenyl, whereby the thiophenyl radical can be substituted with 1, 2 or 3 substituents independently selected from the group consisting of F, Cl, Br, methyl, ethyl and n-propyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, decahydronaphthyl, octahydro-cyclopenta[c]pyrrolyl, decahydroquinolinyl and dodecahydro-carbazolyl,
which may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -0-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, - S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -O$CF_3$, -S$CF_3$, -S$CF_2$H, -SC$FH_2$, -OH, -SH, -$NO_2$, -CHO, -C(=O)-$CH_3$, -C(=O)-$C_2H_5$, -C(=O)-C($CH_3$)$_3$, -$CF_2$H, -CF$H_2$, -C(=O)-$NH_2$, -C(=O)-NH-$CH_3$, - C (=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, - S(=O)-$C_2H_5$, -S(=O)-$C_3H_7$, -S (=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and —N($C_2H_5$)$_2$;
and $R^3$ to $R^{13}$, X, A and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Preference is also given to quaternary ammonium salts of substituted pyrazoline compounds of general formula I, wherein $R^3$ and $R^4$, independently of one another, in each case represent H; F; Cl; Br; I; -CN; -$NO_2$; -NC; -OH; -$NH_2$; -SH; -C(=O)-H;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of—OH, F, Cl, Br, I, -O-$CH_3$, -0-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -$NH_2$, - NH-$CH_3$, -NH-$C_2H_5$, -N($CH_3$)$_2$,—N($C_2H_5$)$_2$, -CN and -$NO_2$;
a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -0-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, - S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -O$CF_3$, -S$CF_3$, -S$CF_2$H, -SC$FH_2$, -OH, -SH, -$NO_2$, -CHO, -C (=O)-$CH_3$, -C(=O)-$C_2H_5$, -C(=O)-C($CH_3$)$_3$, -$CF_2$H, -CF$H_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S (=O)$_2$-$C_3H_7$, -$NH_2$, -NH-$CH_3$, -NH-$C_2H_5$,-N($CH_3$)$_2$ and -N($C_2H_5$)$_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -O$CF_3$, -S$CF_3$, -S$CF_2$H, -SC$FH_2$, -OH, -SH, -$NO_2$, - CHO, -C(=O)-$CH_3$, -C(=O)-$C_2H_5$, -C(=O)-C($CH_3$)$_3$, -$CF_2$H, -CF$H_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S (=O)-$C_3H_7$, -S(=O)$_2$-$CH_3$, -S(=O)$_2$-$C_2H_5$, - S(=O)$_2$-$C_3H_7$, -$NH_2$, -NH-$CH_3$, -NH-$C_2H_5$, -N($CH_3$)$_2$ and -N($C_2H_5$)$_2$;
a -O-$R^7$ moiety; a -S-$R^8$ moiety, a -NH-$R^9$ moiety or a -N$R^{10}R^{11}$ moiety;
and $R^1$, $R^2$, $R^5$ to $R^{13}$, X, A and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Also preferred are quaternary ammonium salts of substituted pyrazoline compounds of general formula I given

above, wherein $R^5$ and $R^6$, independently of one another, in each case represent a hydrogen atom;
or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $-NO_2$;
and $R^1$ to $R^4$, $R^7$ to $R^{13}$, X, A and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Preference is also given to quaternary ammonium salts of substituted pyrazoline compounds of general formula I, wherein $R^5$ and $R^6$ together with the bridging nitrogen atom form a moiety selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (2,5)-dihydro-1H-pyrrolyl, (2,3)-dihydro-1 H-pyrrolyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, indolinyl, isoindolinyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3, 5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl and (1,2,3,4)-tetrahydroquinoxazlinyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $- C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $- OCF_3$, $-SCF_3$, $-SCF_2H$, $-SCFH_2$, -OH, -SH, $-NO_2$, -CHO, $-C(=O)-CH_3$, $-C(=O)-C_2H_5$, $- C(=O)-C(CH_3)_3$, -C(=O)-OH, $-C(=O)-O-CH_3$, $-C(=O)-O-C_2H_5$, $-C(=O)-O-C(CH_3)_3$, $- CF_2H$, $-CFH_2$, $-C(=O)-NH_2$, $-C(=O)-NH-CH_3$, $-C(=O)-NH-C_2H_5$, $-C(=O)-NH-C_3H_7$, $- C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;
and $R^1$ to $R^4$, $R^7$ to $R^{13}$, X, A and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Also preferred are quaternary ammonium salts of substituted pyrazoline compounds of general formula I given above, wherein $R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $- O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $—N(C_2H_5)_2$, -CN and $-NO_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a $-(CH_2)-$, $-(CH_2)-(CH_2)-$, $-(CH_2)-(CH_2)-(CH_2)-$ or-CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $- S-CH(CH_3)_2$, $-S-CH_2-CH_2-CH_2-CH_3$, $-S-C(CH_3)_3$, F, Cl, Br, I, -CN, $-OCF_3$, $-SCF_3$, $- SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $- C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $- S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$,

-CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$,$-N(CH_3)_2$ and $-N(C_2H_5)_2$;
and $R^1$ to $R^6$, $R^{12}$, $R^{13}$, X, A and B have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Preference is also given to quaternary ammonium salts of substituted pyrazoline compounds of general formula I, wherein B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, -OH, $-O-CH_3$ and $-O-C_2H_5$, pyruvate, ascorbate, glycolate, nicotinate, phenylacetate,

$$R^{12}\text{-}SO_3^{\ominus} \quad \text{and} \quad R^{13}\text{-}NH\text{-}SO_3^{\ominus};$$

and $R^1$ to $R^{13}$, X and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Also preferred are quaternary ammonium salts of substituted pyrazoline compounds of general formula I given above, wherein $R^{12}$ and $R^{13}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl, n-tetradecyl, vinyl, n-propenyl, n-butenyl, n-pentenyl, n-hexenyl, ethinyl, propinyl, n-butinyl, n-pentinyl and n-hexinyl; which may optionally be substituted with 1, 2, 3, 4, 5, 6, 7, 8 or 9 substituent(s) independently selected from the group consisting of F, Cl, Br, $-NH_2$, $-NH-CH_3$, $-NH-C_2H_5$, $-N(CH_3)_2$ and $-N(C_2H_5)_2$;
a radical selected from the group consisting of phenyl, naphthyl, pyridinyl, benzimidazolyl, isoquinolinyl and pyrazolyl, which may be bonded via a $-(CH_2)-$, - $(CH_2)-(CH_2)-$ or $-(CH_2)-(CH_2)-(CH_2)-$group and/or may be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-0-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, - $C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-SO_3H$, - $NH_2$, $-NH-CH_3$, $-NH-C_2H_5$, $-N(CH_3)_2$, $-N(C_2H_5)_2$ and phenyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a — $(CH_2)-$, $-(CH_2)-(CH_2)-$, $-(CH_2)-(CH_2)-(CH_2)-$group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, - $O-CH_2-O-C_2H_5$, $-C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, - $C(=O)-C_2H_5$, $-C(=O)-C(CH_3)_3$, $-C(=O)-OH$, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, - $S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and - $N(C_2H_5)_2$;
and $R^1$ to $R^{11}$, X, B and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof.

Preference is also given to quaternary ammonium salts of substituted pyrazoline compounds of general formula I, wherein
X is O or S:
A represents a monovalent oxygen anion ($O^-$) or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $-NO_2$;

$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of $-CF_3$, $-CH_2-Cl$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, - CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, decahydronaphthyl, octahydro-cyclopenta[c]pyrrolyl, decahydroquinolinyl and dodecahydro-carbazolyl,

which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-0-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

$R^3$ and $R^4$, independently of one another, in each case represent H; F; Cl; Br; I; -CN; $-NO_2$; -NC; -OH; $-NH_2$; -SH; -C(=O)-H; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of -OH, F, Cl, Br, I, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, -S-

$CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, - CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $- S(=O)_2-C_3H_7$, $-NH_2$, $-NH-CH_3$, $-NH-C_2H_5$, $-N(CH_3)_2$ and $-N(C_2H_5)_2$;

a —O-$R^7$ moiety; a -S-$R^8$ moiety, a -NH-$R^9$ moiety or a —$NR^{10}R^{11}$ moiety;

$R^5$ and $R^6$, independently of one another, in each case represent a hydrogen atom;

or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $-NO_2$;

or

$R^5$ and $R^6$ together with the bridging nitrogen atom form a moiety selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (2,5)-dihydro-1 H-pyrrolyl, (2,3)-dihydro-1 H-pyrrolyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, indolinyl, isoindolinyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl and (1,2,3,4)-tetrahydroquinoxazlinyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $- C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $- OCF_3$, $-SCF_3$, $-SCF_2H$, $-SCFH_2$, -OH, -SH, $-NO_2$, -CHO, $-C(=O)-CH_3$, $-C(=O)-C_2H_5$, $- C(=O)-C(CH_3)_3$, -C(=O)-OH, $-C(=O)-O-CH_3$, $-C(=O)-O-C_2H_5$, $-C(=O)-O-C(CH_3)_3$, $- CF_2H$, $-CFH_2$, $-C(=O)-NH_2$, $-C(=O)-NH-CH_3$, $-C(=O)-NH-C_2H_5$, $-C(=O)-NH-C_3H_7$, $- C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

$R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $— N(C_2H_5)_2$, -CN and $-NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $- S-CH(CH_3)_2$, $-S-CH_2-CH_2-CH_2-CH_3$, $-S-C(CH_3)_3$, F, Cl, Br, I, -CN, $-OCF_3$, $-SCF_3$, $- SCF_2H$, $-SCFH_2$, -OH, -SH, $-NO_2$. -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $- C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $- S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, -S

$(=O)_2$-$C_2H_5$, -$S(=O)_2$-$C_3H_7$, -$NH_2$, -NH-$CH_3$, -NH-$C_2H_5$, - $N(CH_3)_2$ and —$N(C_2H_5)_2$;
and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, -OH, -O-$CH_3$ and -O-$C_2H_5$, pyruvate, ascorbate, glycolate, nicotinate, phenylacetate,

$$R^{12}\text{-}SO_3^{\ominus} \quad \text{and} \quad R^{13}\text{-NH-}SO_3^{\ominus};$$

and
$R^{12}$ and $R^{13}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl, n-tetradecyl, vinyl, n-propenyl, n-butenyl, n-pentenyl, n-hexenyl, ethinyl, propinyl, n-butinyl, n-pentinyl and n-hexinyl; which may optionally be substituted with 1, 2, 3, 4, 5, 6, 7, 8 or 9 substituent(s) independently selected from the group consisting of F, Cl, Br, - $NH_2$, -NH-$CH_3$, -NH-$C_2H_5$, -$N(CH_3)_2$ and -$N(C_2H_5)_2$;
a radical selected from the group consisting of phenyl, naphthyl, pyridinyl, benzimidazolyl, isoquinolinyl and pyrazolyl, which may be bonded via a -($CH_2$)-, - ($CH_2$)-($CH_2$)- or -($CH_2$)-($CH_2$)-($CH_2$)-group and/or may be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -0-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, - C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$SO_3H$, - $NH_2$, -NH-$CH_3$, -NH-$C_2H_5$, -$N(CH_3)_2$, -$N(C_2H_5)_2$ and phenyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a — ($CH_2$)-, -($CH_2$)-($CH_2$)-, -($CH_2$)-($CH_2$)-($CH_2$)-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -0-$CH(CH_3)_2$, -O-$CH_2$-$CH_2$-$CH_2$-$CH_3$, -O-C $(CH_3)_3$, -O-$CH_2$-O-$CH_3$, -O-$CH_2$-$CH_2$-O-$CH_3$, - O-$CH_2$-O-$C_2H_5$, -$C(OCH_3)(C_2H_5)_2$, -$C(OCH_3)(CH_3)_2$, -$CH_2$-O-$CH_3$, -$CH_2$-O-$C_2H_5$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, - C(=O)-$C_2H_5$, -C(=O)-$C(CH_3)_3$, -C(=O)-OH, -C (=O)-O-$CH_3$, -C(=O)-O-$C_2H_5$, -C(=O)-O-$C(CH_3)_3$, -$CF_2H$, -$CFH_2$, -C(=O)-$NH_2$, -C(=O)-NH-$CH_3$, -C(=O)-NH-$C_2H_5$, -C (=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, - $S(=O)_2$-$CH_3$, -S $(=O)_2$-$C_2H_5$, -$S(=O)_2$-$C_3H_7$, -$NH_2$, -NH-$CH_3$, -NH-$C_2H_5$, -$N(CH_3)_2$ and — $N(C_2H_5)_2$.
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

Particularly preferred are quaternary ammonium salts of substituted pyrazoline compounds of general formula I given above, wherein
X is O or S;
A represents a monovalent oxygen anion ($O^-$) or a radical selected from the group consisting of methyl, ethyl, n-propyl, isopropyl, n-butyl, isobutyl, sec-butyl and tert-butyl;
$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of -$CF_3$, -$CH_2$-Cl, -0-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, methyl, ethyl, n-propyl, isopropyl, n-butyl, isobutyl, 2-butyl, tert-butyl, n-pentyl, 2-pentyl, n-hexyl, F, Cl, Br, I, -CN, -$OCF_3$, -$SCF_3$, -$SCF_2H$,

-SCFH$_2$, -OH, -SH, -NO$_2$, - CHO, -CF$_2$H, -CFH$_2$, phenoxy and thiophenyl, whereby the thiophenyl radical can be substituted with 1, 2 or 3 substituents independently selected from the group consisting of F, Cl, Br, methyl, ethyl and n-propyl;

$R^3$ represents H; F; Cl; Br; I; -CN; -NO$_2$; -NC; -OH; -NH$_2$; -SH; -C(=O)-H;

a radical selected from the group consisting of methyl, -CF$_3$, -CH$_2$F, -CF$_2$H, -C$_2$F$_5$, ethyl, -CH$_2$-CN, -CH$_2$-OH, n-propyl, isopropyl, -CH$_2$-CH$_2$-CN, -CH$_2$-CH$_2$-OH, n-butyl, - CH$_2$-CH$_2$-CH$_2$-CN, -CH$_2$-CH$_2$-CH$_2$-OH, isobutyl, sec-butyl, tert-butyl, n-pentyl, 2-pentyl, 3-pentyl, neo-pentyl, n-hexyl, 2-hexyl and 3-hexyl;

a radical selected from the group consisting of phenyl, naphthyl, pyridinyl, furyl (furanyl), thienyl (thiophenyl) and pyrrolyl, which may be bonded via a —(CH$_2$)-group and/or may optionally be substituted with 1, 2, 3, 4 or 5 substituent(s) independently selected from the group consisting of —CF$_3$, methyl, ethyl, n-propyl, isopropyl, n-butyl, isobutyl, 2-butyl, tert-butyl, n-pentyl, 2-pentyl, n-hexyl, -O-CH$_3$, -O-C$_2$H$_5$, F, Cl and Br;

a —O-$R^7$ moiety; a —S-$R^8$ moiety, a —NH-$R^9$ moiety or a —N$R^{10}R^{11}$ moiety;

$R^4$ represents H or an alkyl radical selected from the group consisting of methyl, ethyl, n-propyl, isopropyl and n-butyl;

$R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl and n-pentyl;

$R^5$ and $R^6$ together with the bridging nitrogen atom form a moiety selected from the group consisting of

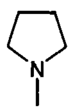 , 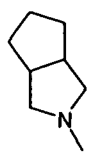 , 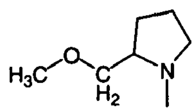 , 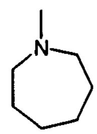 , 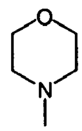 , 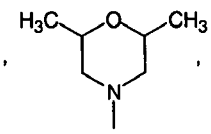 ,

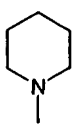 , 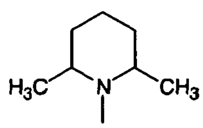 , 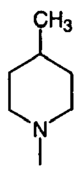 , 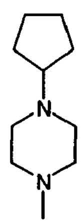 , 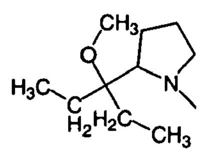 , 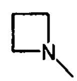 ,

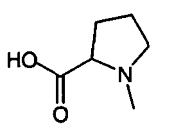 , 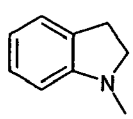 , 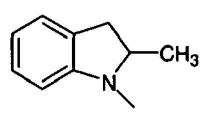 , 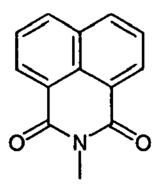 , 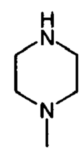 ,

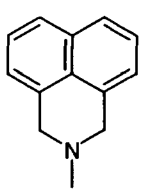 , 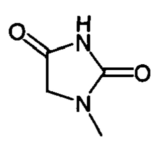 , 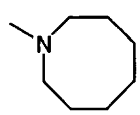 , 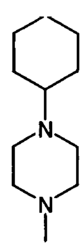 , 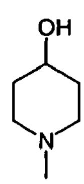 , 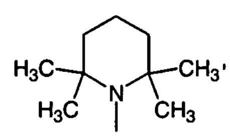 ,

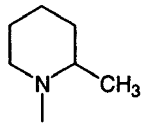, 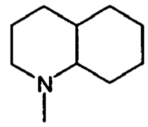, 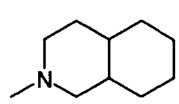, 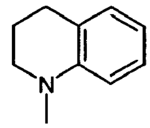, 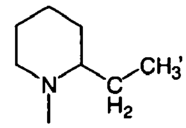

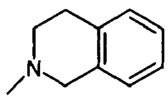, 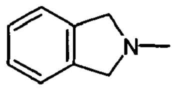, 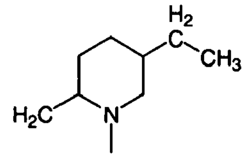 and 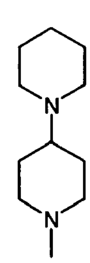 ;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, -OH, -O-$CH_3$ and -O-$C_2H_5$ , pyruvate, ascorbate, glycolate, nicotinate, phenylacetate,

$$R^{12}\text{-}SO_3^{\ominus} \quad \text{and} \quad R^{13}\text{-}NH\text{-}SO_3^{\ominus};$$

and
$R^{12}$ and $R^{13}$, independently of one another, in each case represent a radical selected from the group consisting of —$CF_3$, -$C_2F_5$, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -0-C($CH_3$)$_3$, -OH, -$NO_2$, -$NH_2$, phenyl and -$SO_3$H;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a — ($CH_2$)-, -($CH_2$)-($CH_2$)-, -($CH_2$)-($CH_2$)-($CH_2$)-group;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

More particularly preferred are quaternary ammonium salts of substituted pyrazoline compounds of general formula I given above, wherein
X is O;
A represents a monovalent oxygen anion (O') or a radical selected from the group consisting of methyl, ethyl and n-propyl;
$R^1$ represents a radical selected from the group consisting of phenyl and thienyl (thiophenyl), which may optionally be substituted with 1, 2 or 3 substituent(s) independently selected from the group consisting of F, Cl, Br, I, -O-$CH_3$ and -O-$C_2H_5$;
$R^2$ represents a phenyl radical, which may be substituted with 1, 2 or 3 substituent(s) independently selected from the group consisting of F, Cl, Br and I;
$R^3$ represents H; F; Cl; Br; I; -OH, -CN; -C(=O)-H;
a radical selected from the group consisting of methyl, -$CF_3$, -$CH_2$F, -$CF_2$H, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl, - $CH_2$-$CH_2$-$CH_2$-CN, -$CH_2$-$CH_2$-$CH_2$-OH, isobutyl, sec-butyl, tert-butyl,

n-pentyl, 2-pentyl, 3-pentyl, neo-pentyl, n-hexyl, 2-hexyl and 3-hexyl;
a benzyl radical or a -O-$R^7$ moiety;
$R^4$ represents H or a radical selected from the group consisting of methyl, ethyl, n-propyl, iso-propyl and n-butyl;
$R^5$ and $R^6$ together with the bridging nitrogen atom form a moiety selected from the group consisting of

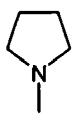, 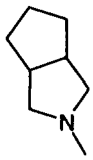, 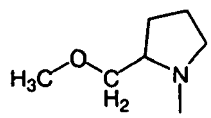, 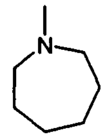, 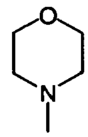, 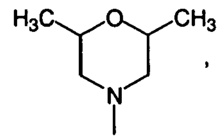,

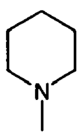, 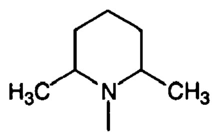, 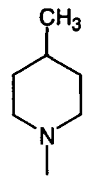, 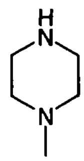, 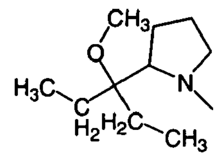,

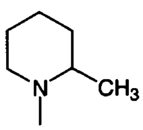, 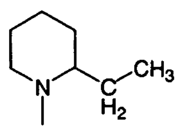, 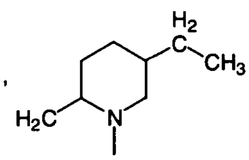, 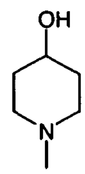 and 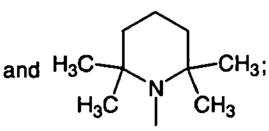;

$R^7$ represents a radical selected from the group consisting of methyl, ethyl, n-propyl, isopropyl, n-butyl, isobutyl, sec-butyl, tert-butyl and n-pentyl;
and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfo-napthalene-1-sulfonate, cyclamate, dodecane-1-sulfonate and (7,7)-dimethyl-2-oxo-bicyclo [2.2.1]-hept-1-yl-methanesulfonate;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

Most particularly preferred are quaternary ammonium salts of substituted pyrazoline compounds of general formula I given above selected from the group consisting of

[1] 1-[[5-(4-Chloro-phenyl)-1-(2,4-dichloro-phenyl)-4,5-dihydro-1H-pyrazole-3-carbonyl]-amino]-1-methyl-piperidinium iodide

[2] 1-[[5-(4-Chtoro-phenyl)-1-(2,4-dichloro-phenyl)-4,5-dihydro-1H-pyrazole-3-carbonyl]-amino]-4-methyl-morpholin-4-ium iodide

[3] 1-[[5-(4-Chloro-phenyl)-1-(2,4-dichloro-phenyl)-4,5-dihydro-1H-pyrazole-3-carbonyl]-amino]-1-methyl-azepanium iodide

[4] 1-[[5-(4-Chloro-phenyl)-1-(2,4-dichloro-phenyl)-4,5-dihydro-1H-pyrazole-3-carbonyl]-amino]-2-methyl-octahydro-cyclopenta[c]pyrrolium iodide

[5] 1-[[5-(4-Chloro-phenyl)-1-(2,4-dichloro-phenyl)-4,5-dihydro-1H-pyrazole-3-carbonyl]-amino]-1-methyl-pyrrolidinium iodide

[6] (R)-1-[[5-(4-Chloro-phenyl)-1-(2,4-dichloro-phenyl)-4,5-dihydro-1H-pyrazole-3-carbonyl]-amino]-1-methyl-piperidinium iodide

[7] (S)-1-[[5-(4-Chloro-phenyl)-1-(2,4-dichloro-phenyl)-4,5-dihydro-1H-pyrazole-3-carbonyl]-amino]-1-methyl-piperidinium iodide

[8] 1-[[5-(4-Chloro-phenyl)-1-(2,4-dichloro-phenyl)-4,5-dihydro-1H-pyrazole-3-carbonyl]-amino]-1-ethyl-piperidinium iodide

[9] (R)-1-[[5-(4-Chloro-phenyl)-1-(2,4-dichloro-phenyl)-4,5-dihydro-1 H-pyrazole-3-carbonyl]-amino]-1-ethyl-piperidinium iodide

[10] (S)-1-[[5-(4-Chloro-phenyl)-1-(2,4-dichloro-phenyl)-4,5-dihydro-1H-pyrazole-3-carbonyl]-amino]-1-ethyl-piperidinium iodide

[11] 1-[[5-(4-Bromo-phenyl)-1-(2,4-dichloro-phenyl)-4,5-dihydro-1H-pyrazole-3-carbonyl]-amino]-1-methyl-piperidinium iodide

[12] 1-[[2,4-Dichloro-phenyl)-5-(4-iodo-phenyl)-4,5-dihydro-1H-pyrazole-3-carbonyl]-amino]-1-methyl-piperidinium iodide

[13] 1-[[2,4-Dichloro-phenyl)-5-(4-fluoro-phenyl)-4,5-dihydro-1H-pyrazole-3-carbonyl]-amino]-1-methyl-piperidinium iodide

[14] 1-[[2,4-Dichloro-phenyl)-5-(4-methoxy-phenyl)-4,5-dihydro-1H-pyrazole-3-carbonyl]-amino]-1-methyl-piperidinium iodide

[15] 1-[[2,4-Dichloro-phenyl)-5-(4-methoxy-phenyl)-4,5-dihydro-1H-pyrazole-3-carbonyl]-amino]-1-methyl-azepanium iodide

[16] 1-[[2,4-Dichloro-phenyl)-5-(4-fluoro-phenyl)-4,5-dihydro-1H-pyrazole-3-carbonyl]-amino]-1-methyl-azepanium iodide

[17] 1-[[2,4-Dichioro-phenyl)-5-(4-iodo-phenyl)-4,5-dihydro-1H-pyrazole-3-carbonyl]-amino]-1-methyl-azepanium iodide

[18] 1-[[5-(4-Bromo-phenyl)-1-(2,4-dichloro-phenyl)-4,5-dihydro-1H-pyrazole-3-carbonyl]-amino]-1-methyl-azepanium iodide

[19] 1-[[5-(4-Bromo-phenyl)-1-(2,4-dichloro-phenyl)-4,5-dihydro-1H-pyrazole-3-carbonyl]-amino]-1-methyl-pyrrolidinium iodide

[20] 1-[[2,4-Dichloro-phenyl)-5-(4-fluoro-phenyl)-4,5-dihydro-1H-pyrazole-3-carbonyl]-amino]-1-methyl-pyrrolidinium iodide

[21] 1-[[2,4-Dichloro-phenyl)-5-(4-methoxy-phenyl)-4,5-dihydro-1H-pyrazole-3-carbonyl]-amino]-1-methyl-pyrrolidinium iodide

[22] cis-1-[[5-(4-Chloro-phenyl)-1-(2,4-dichloro-phenyl)-4-methyl-4,5-dihydro-1H-pyrazole-3-carbonyl]-amino]-1-methyl-piperidinium iodide

[23] cis-1-[[5-(4-Chloro-phenyl)-1-(2,4-dichloro-phenyl)-4-ethyl-4,5-dihydro-1H-pyrazole-3-carbonyl]-amino]-1-methyl-piperidinium iodide

[24] cis-1-[[5-(4-Bromo-phenyl)-1-(2,4-dichloro-phenyl)-4-methyl-4,5-dihydro-1H-pyrazole-3-carbonyl]-amino]-1-methyl-piperidinium iodide

[25] cis-1-[[5-(4-Fluoro-phenyl)-1-(2,4-dichloro-phenyl)-4-ethyl-4,5-dihydro-1H-pyrazole-3-carbonyl]-amino]-1-methyl-piperidinium iodide

[26] trans-1-[[5-(4-Chloro-phenyl)-1-(2,4-dichloro-phenyl)-4-methyl-4,5-dihydro-1H-pyrazole-3-carbonyl]-amino]-1-methyl-piperidinium iodide

[27] trans-1-[[5-(4-Chloro-phenyl)-1-(2,4-dichloro-phenyl)-4-ethyl-4,5-dihydro-1H-pyrazole-3-carbonyl]-amino]-1-methyl-piperidinium iodide

[28] trans-1-[[5-(4-Bromo-phenyl)-1-(2,4-dichloro-phenyl)-4-methyl-4,5-dihydro-1H-pyrazole-3-carbonyl]-amino]-1-methyl-piperidinium iodide

[29] trans-1-[[5-(4-Fluoro-phenyl)-1-(2,4-dichloro-phenyl)-4-ethyl-4,5-dihydro-1H-pyrazole-3-carbonyl]-amino]-1-methyl-piperidinium iodide

[30] cis-1-[[5-(4-Chloro-phenyl)-1-(2,4-dichloro-phenyl)-4-methyl-4,5-dihydro-1H-pyrazole-3-carbonyl]-amino]-1-methyl-azepanium iodide

[31] cis-1-[[5-(4-Chioro-phenyl)-1-(2,4-dichioro-phenyl)-4-methyl-4,5-dihydro-1H-pyrazole-3-carbonyl]-amino]-1-methyl-pyrrolidinium iodide

[32] trans-1-[[5-(4-Chloro-phenyl)-1-(2,4-dichloro-phenyl)-4-methyl-4,5-dihydro-1H-pyrazole-3-carbonyl]-amino]-1-methyl-pyrrolidinium iodide

[33] trans-1-[[5-(4-Chloro-phenyl)-1-(2,4-dichloro-phenyl)-4-methyl-4,5-dihydro-1H-pyrazole-3-carbonyl]-amino]-1-methyl-azepanium iodide

[34] cis-2-[[5-(4-Chloro-phenyl)-1-(2,4-dichioro-phenyl)-4-methyl-4,5-dihydro-1H-pyrazole-3-carbonyl]-amino]-2-methyl-octahydrocyclopenta[c]pyrrolium iodide

[35] trans-2-[[5-(4-Chloro-phenyl)-1-(2,4-dichloro-phenyl)-4-methyl-4,5-dihydro-1H-pyrazole-3-carbonyl]-amino]-2-methyl-octahydrocyclopenta[c]pyrrolium iodide

[36] 2-[[5-(4-Chloro-phenyl)-1-(2,4-dichloro-phenyl)-4,4-dimethyl-4,5-dihydro-1H-pyrazole-3-carbonyl]-amino]-2-methyl-octahydrocyclopenta[c]pyrrolium iodide

[37] 2-[[5-(4-Chloro-phenyl)-1-(2,4-dichloro-phenyl)-4,4-dimethyl-4,5-dihydro-1H-pyrazole-3-carbonyl]-amino]-1-methyl-piperidinium iodide

[38] cis-1-[[5-(4-Chloro-phenyl)-1-(2,4-dichloro-phenyl)-4-trifluormethyl-4,5-dihydro-1H-pyrazole-3-carbonyl]-amino]-1-methyl-piperidinium iodide

[39] cis-1-[[5-(4-Chloro-phenyl)-1-(2,4-dichloro-phenyl)-4-methoxy-4,5-dihydro-1H-pyrazole-3-carbonyl]-amino]-1-methyl-piperidinium iodide

[40] cis-1-[[5-(4-Chloro-phenyl)-1-(2,4-dichloro-phenyl)-4-cyano-4,5-dihydro-1H-pyrazole-3-carbonyl]-amino]-1-methyl-piperidinium iodide

[41] 1-[[5-(5-Chloro-thiophen-2-yl)-1-(2,4-dichioro-phenyl)-4,5-dihydro-1H-pyrazole-3-carbonyl]-amino]-1-methyl-piperidinium iodide

[42] 1-[[5-(5-Bromo-thiophen-2-yl)-1-(2,4-dichloro-phenyl)-4,5-dihydro-1H-pyrazole-3-carbonyl]-amino]-1-methyl-piperidinium iodide

[43] 1-[[5-(4-Bromo-thiophen-2-yl)-1-(2,4-dichioro-phenyl)-4,5-dihydro-1H-pyrazole-3-carbonyl]-amino]-1-methyl-piperidinium iodide

[44] cis-1-[[5-(5-Chloro-thiophen-2-yl)-1-(2,4-dichloro-phenyl)-4-methyl-4,5-dihydro-1 H-pyrazole-3-carbonyl]-amino]-1-methyl-piperidinium iodide

[45] trans-1-[[5-(5-Chloro-thiophen-2-yl)-1-(2,4-dichloro-phenyl)-4-methyl-4,5-dihydro-1H-pyrazole-3-carbonyl]-amino]-1-methyl-piperidinium iodide

[46] cis-1-[[5-(5-Bromo-thiophen-2-yl)-1-(2,4-dichloro-phenyl)-4-methyl-4,5-dihydro-1 H-pyrazole-3-carbonyl]-amino]-1-methyl-piperidinium iodide

[47] trans-1-[[5-(5-Bromo-thiophen-2-yl)-1-(2,4-dichloro-phenyl)-4-methyl-4,5-dihydro-1H-pyrazole-3-carbonyl]-amino]-1-methyl-piperidinium iodide

[48] cis-1-[[5-(4-Bromo-thiophen-2-yl)-1-(2,4-dichloro-phenyl)-4-methyl-4,5-dihydro-1 H-pyrazole-3-carbonyl]-amino]-1-methyl-piperidinium iodide

[49] trans-1-[[5-(4-Bromo-thiophen-2-yl)-1-(2,4-dichloro-phenyl)-4-methyl-4,5-dihydro-1H-pyrazole-3-carbonyl]-amino]-1-methyl-piperidinium iodide and

[50] cis-1-[[5-(4-Bromo-thiophen-2-yl)-1-(2,4-dichloro-phenyl)-4-hydroxy-4-methyl-4,5-dihydro-1H-pyrazole-3-carbonyl]-amino]-1-methyl-piperidinium iodide optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

In another aspect the present invention also provides a process for the preparation of a salt of general formula I given above, wherein $R^4$ represents hydrogen,
according to which at least one compound of general formula $R^1$-C(=O)-H (general formula II), wherein $R^1$ has the meaning given above, is reacted with at least one compound of general formula III,

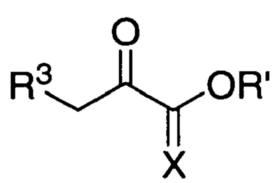

**III,**

wherein $R^3$ and X have the meaning given above and R' represents a linear or branched $C_{1-6}$-alkyl radical, a potassium cation or a sodium cation, in a reaction medium, optionally in an inert atmosphere, optionally in the presence of at least one base, to yield at least one compound of general formula IV,

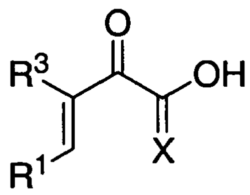

IV,

wherein $R^1$, X and $R^3$ have the meaning given above, which is optionally purified and/or isolated,
and at least one compound of general formula IV is reacted with at least one compound of general formula V,

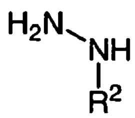

V,

or a corresponding salt thereof, wherein $R^2$ has the meaning given above, in a reaction medium, optionally in an inert atmosphere, optionally in the presence of at least one acid, to yield at least one compound of general formula VI,

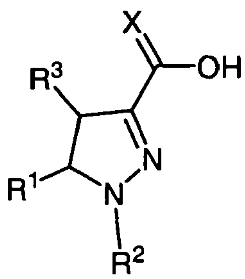

VI

wherein $R^1$, X, $R^2$ and $R^3$ have the meaning given above, which is optionally isolated and/or purified,
and at least one compound of general formula VI is reacted with an activating agent in a reaction medium, optionally in an inert atmosphere, to yield at least one compound of general formula VII,

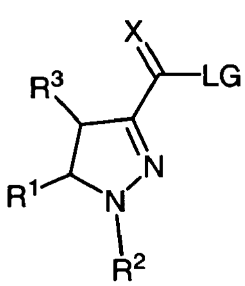

VII

wherein $R^1$, X, $R^2$ and $R^3$ have the meaning given above and LG represents a leaving group, which is optionally purified and/or isolated,
and at least one compound of general formula VII is reacted with at least one compound of general formula $NH_2$-$NR^5R^6$, wherein $R^5$ and $R^6$ have the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula VIII,

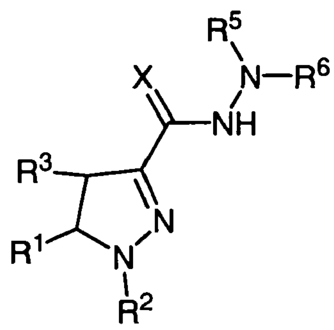

VIII

wherein $R^1$, $R^2$, $R^3$, X, $R^5$ and $R^6$ have the meaning given above, which is optionally purified and/or isolated;
or at least one compound of general formula VI is reacted with at least one compound of general formula $NH_2$-$NR^5R^6$, wherein $R^5$ and $R^6$ have the meaning given above, in a reaction medium, in the presence of at least one coupling agent, optionally in the presence of at least one base, to yield at least one compound of general VIII, which is optionally purified and/or isolated;
and at least one compound of general formula VIII is reacted with at least one compound of general formula A-B, wherein A and B have the meaning given above, in a reaction medium, to yield at least one salt of general formula I,

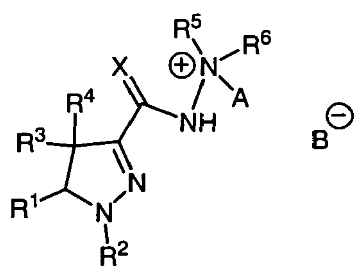

I

wherein A, B, $R^1$, $R^2$, $R^3$, X, $R^5$ and $R^6$ have the meaning given above and $R^4$ represents hydrogen, which is optionally purified and/or isolated.
The inventive process is also illustrated in scheme I given below:

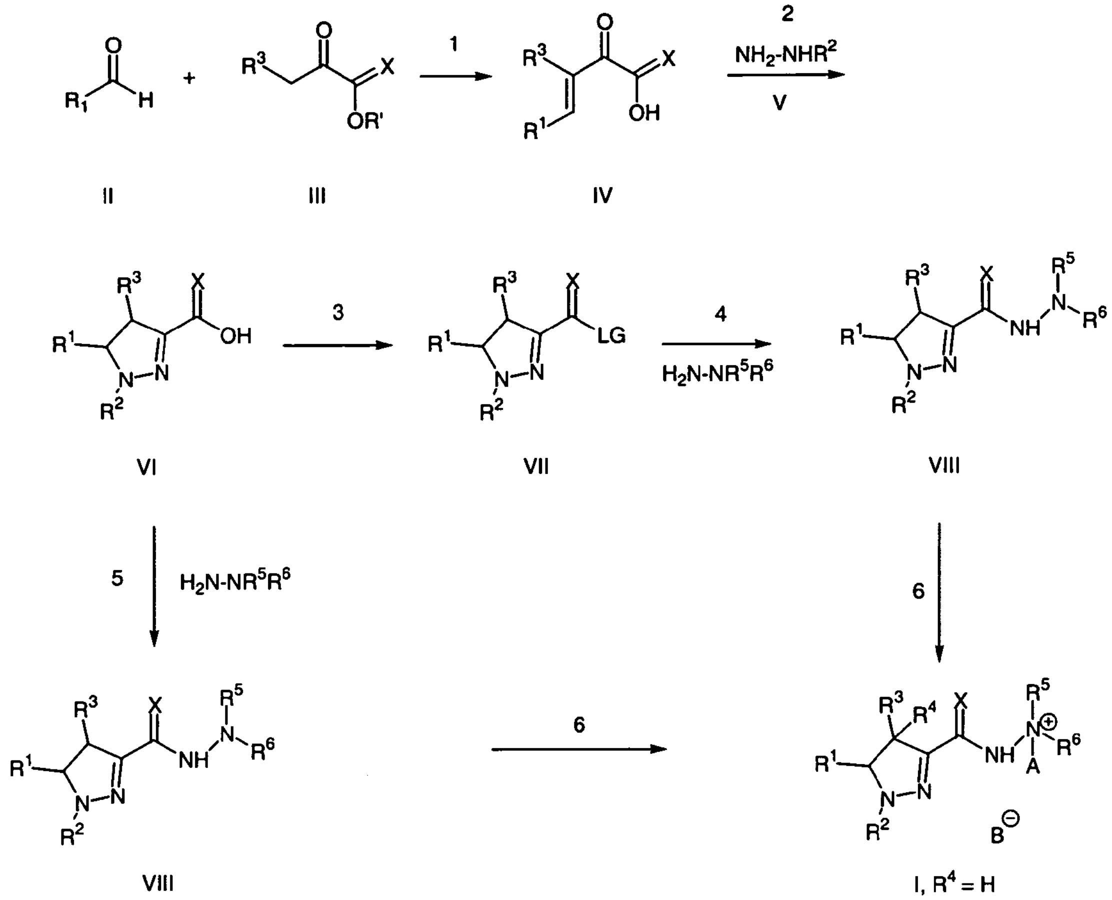


Scheme I

In step 1 a compound of general formula II is reacted with a compound of general formula III in a protic reaction medium, preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide, as described, for example, in Synthetic Communications, 26(11), 2229-33, (1996). The respective description is hereby incorporated by reference and forms part of the disclosure. Reaction temperature as well as the duration of the reaction may vary over a broad range. Preferred reaction temperatures range from -10 °C to the boiling point of the reaction medium. Suitable reaction times may vary for example from several minutes to several hours.

Preferably the reaction between a compound of general formula II and a compound of general formula III can also be carried out under acid catalysed conditions, more preferably by refluxing the above mentioned compounds in dichloromethane in the presence of copper(II)trifluoromethanesulfonate as described, for example, in Synlett, (1), 147-149, 2001. The respective description is hereby incorporated by reference and forms part of the disclosure.

In step 2 a compound of general formula IV is reacted with a compound of general formula V in a reaction medium, preferably in a reaction medium selected from the group consisting of methanol, ethanol, isopropanol, n-butanol, dieethylether, tert-butyl-methylether, dioxane, tetrahydrofurane or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula VI may be activated for further reactions by the introduction of a suitable leaving group according to conventional methods well known to those skilled in the art. Preferably the compounds of general formula VI are transferred into an acid chloride, an acid anhydride, a mixed anhydride, a $C_{1-4}$

alkyl ester or an activated ester such as p-nitrophenylester. Suitable activating agent therefore are selected from the group consisting of thionyl chloride, oxalyl chloride and ethylchloroformate.

If said activated compound of general formula VII is an acid chloride, wherein LG represents a chlorine atom, that compound is preferably prepared by the reaction of the corresponding acid of general formula VI with thionyl chloride or oxalyl chloride, whereby said chlorinating agent is also used as the reaction medium, in the presence of at least one base, preferably in the presence of a base selected from the group consisting of triethylamine, N-methylmorpholine, pyridine, dimethylaminopyridine and diisopropylethylamine. Also preferably an additional reaction medium may be used. Suitable reaction media include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane or dimethylformamide and mixtures thereof. More preferably toluene in the presence of a catalytic amount of dimethylformamide is used as reaction medium. Preferred reaction temperature range from 0 C to the boiling point of the solvent and reaction times vary from several minutes to several hours.

If said activated compound of general formula VII is a mixed anhydride, wherein LG represents -O-C(=O)-O-$C_2H_5$, said anhydride may preferably be prepared, for example, by reaction of the corresponding acid of general formula VI with ethylchloroformate in the presence of a base such as triethylamine, pyridine or diisopropylethylamine, in a suitable solvent such as dichloromethane, optionally in an inert atmosphere, at a temperature between -50˚C and 50 ˚C.

In step 4 the reaction between a compound of general formula VII with a compound of general formula $NH_2$-$NR^5R^6$ to yield a compound of general formula VIII is preferably carried out in the presence of a base such as triethylamine in a reaction medium such as methylenchloride. The temperature is preferably in the range from 0 C to the boiling point of the reaction medium. The reaction time may vary over a broad range, e.g. from several hours to several days.

In step 5 a compound of general formula VI is reacted with a compound of general formula $NH_2$-$NR^5R^6$ in a reaction medium, preferably in a reaction medium selected from the group consisting of diethylether, tetrahydrofuran, acetonitrile, methanol, ethanol, (1,2)-dichlorethane, dimethylformamide, dichlormethane and mixtures thereof, in the presence of at least one coupling agent, preferably in the presence of at least one coupling agent selected from the group consisting of 1-benzotriazolyloxytris-(dimethylamino)-phosphonium hexafluorophosphate (BOP), dicyclohexylcarbodiimide (DCC), N'-(3-dimethylaminopropyl)-N-ethylcarbodiimide (EDCI), diisoproylcarbodiimide, 1,1'-carbonyl-diimidazole (CDI), N-[(dimethyamino)-1 H-1, 2, 3-triazolo[4, 5-b]pyridino-1-ylmethylen]-N-methylmethanaminium hexafluorophosphate N-oxid (HATU), O-(benzotriazol-1-yl)-N,N,N',N'-tetramethyluroniom hexafluorophosphate (HBTU), O-(benzotriazol-1-yl)-N,N,N',N'-tetramethyluronium-tetrafluoroborate (TBTU), 1-hydroxy-benzotriazole (HOBt) and 1-hydroxy-7-azabenzotriazol (HOAt), optionally in the presence of a base, preferably in the presence of at least one base selected from the group consisting of pyridine, dimethylaminopyridine, N-methylmorpholine, triethylamine and diisopropylethylamine to yield a compound of general formula I.

Preferably said reaction is carried out in the presence of EDCI and HOBt, optionally in the presence of N-methylmorpholine or triethylamine, in an aprotic reaction medium such as dimethylformamide or tetrahydrofurane, at a temperature between 20 ˚C and 30 ˚C for 15 to 24 hours as described in Tetrahedron Lett. 2004, 45, 4977. The respective description is hereby incorporated by reference and forms part of the disclosure. Polymer-supported EDCI (P-EDCI) can also suitably be used for this process instead of EDCI as described in Tetrahedron Lett. 1998, 39, 1487 and Tetrahedron Lett. 2002, 43, 7685. The respective descriptions are hereby incorporated by reference and form part of the disclosure.

Alternatively said reaction can be carried out by using HBTU in the presence of a base such as diisopropylethylamine in an aprotic solvent, such as acetonitrile, preferably at a temperature between 20 and 30 ˚C for 15 to 24 hours.

In step 6 a compound of general formula VIII or a compound of general formula IX,

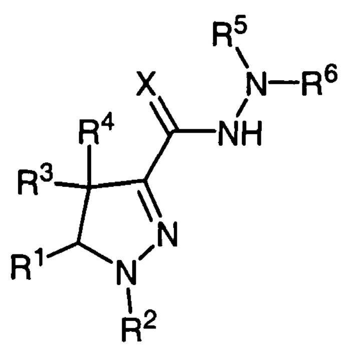

IX

wherein $R^1$, $R^2$, $R^3$, $R^4$, $R^5$, $R^6$ and X have the meaning given above, is reacted with a compound of general formula A-B, wherein A represents an unsubstituted or at least mono-substituted alkyl radical, preferably an unsubstituted $C_{1-10}$ alkyl radical and B represents an anion selected from the group consisting of fluoride, chloride, bromide and iodide, in a reaction medium, preferably in a reaction medium selected from the group consisting of chloroform, acetone, acetonitrile, dichloromethane, carbon tetrachloride, toluene and mixtures of the afore mentioned reaction media, at a temperature between 0 ˚C to 120 ˚C, preferably at a temperature between 20 ˚C to 100 ˚C, to afford a salt of general formula I. The duration of the reaction may vary over a broad range. Suitable reaction times may vary for example from several minutes to several hours.

Alternatively, a compound of general formula VIII or IX given above is reacted with an acid of general formula A-B, wherein A represents an unsubstituted or at least mono-substituted alkyl radical, preferably an unsubstituted $C_{1-10}$alkyl radical, and B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, unsubstituted or at least mono-substituted benzoate or naphthoate, pyruvate, ascorbate, glycolate, nicotinate, phenylacetate,

$$R^{12}\text{-}SO_3^{\ominus} \quad \text{and} \quad R^{13}\text{-NH-}SO_3^{\ominus};$$

wherein $R^{12}$ and $R^{13}$ have the meaning given above; in a reaction medium, preferably in a reaction medium selected from the group consisting of ethyl acetate, chloroform, acetone, acetonitrile, dichloromethane, carbon tetrachloride, toluene, methanol, ethanol, 2-propanol, (1,4)-dioxane, tetrahydrofuran and mixtures of the afore mentioned reaction media, at a temperature between 0 ˚C and 50 ˚C, preferably at a temperature between 5 ˚C and 40 ˚C, for less than an hour, preferably for less than 30 minutes, more preferably for less than 10 minutes, to afford a salt of general formula I.

Suitable acids include hydrochloric, hydrofluoric, hydroiodic or hydrobromic acid; sulfuric, phosphoric, nitric or perchloric acid; thiocyanic acid; cyanic acid; cyclamic acid; aliphatic or aromatic carboxylic or sulfonic acids, e.g. formic, acetic, propionic, succinic, glycolic, lactic, malic, tartaric, gluconic, citric, ascorbic, maleic, fumaric, pyruvic, phenylacetic, benzoic, 4-aminobenzoic, anthranilic, 4-hydroxybenzoic, salicylic, 4-aminosalicylic, nicotinic, methanesulfonic, ethanesulfonic, hydroxyethanesulfonic, ethane-1 ,2-disulfonic, benzenesulfonic, p-toluenesulfonic, naphthalene-2-sulfonic, hydroquinonesulfonic, camphersulfonic, naphthalene-1 ,5-disulfonic, n-dodecyl-sulfonic, sulfanilic or cyclohexylsulfamic acid.

Suitable acids also include 3-amino-1-propanesulfonic acid, aniline-2-sulfonic acid, 3-pyridinesulfonic acid, 4-amino-3-hydroxy-1-naphthalenesulfonic acid, 2-amino-1,5-naphthalenedisulfonic acid, aminomethanesulfonic acid, 2-mesitylenesulfonic acid, 7-amino-1,3-naphthalenedisulfonic acid, (2,5)-diaminobenzenesulfonic acid, trifluormethanesulfonic acid, 1-naphthalenesulfonic acid, 6-amino-m-toluenesulfonic acid, 2-phenylbenzimidazole-5-sulfonic acid, 1-amino-benzimidazole-2-sulfonic acid, 1 H-benzimidazole-2-sulfonic acid, 1-methylbenzimidazole-2-sulfonic acid, 5-isoquinolinesulfonic acid, 3-amino-4-chlorobenzenesulfonic acid, 3-amino-4-methoxybenzenesulfonic acid, 2-amino-5-methoxy-benzenesulfonic acid, 2,4-diamino-benzenesulfonic acid, (3,5)-diamino-2,4,6-trimethylbenzenesulfonic acid, (2,5)-disulfoaniline, 2-aminopropyl-sulfonic acid, 4-amino-1-naphthalenesulfonic acid, 8-amino-1-naphthol-5-sulfonic acid, metanilic acid, 5-chloro-1,3-dimethyl-1H-pyrazole-4-sulfonic acid, 8-chloronaphthalene-1-sulfonic acid, (2,5)-dihydroxybenzenesulfonic acid and 4-amino-3-nitrobenzene-1-sulfonic acid.

Hydrochloric acid is preferably used as a methanolic, ethanolic, ethereal or aqueous solution. Alternatively, hydrogen chloride gas can be used for the preparation of the salts of general formula I given above.

In case $R^4$ is unlike hydrogen the reaction sequence given in Scheme I is adapted as follows.

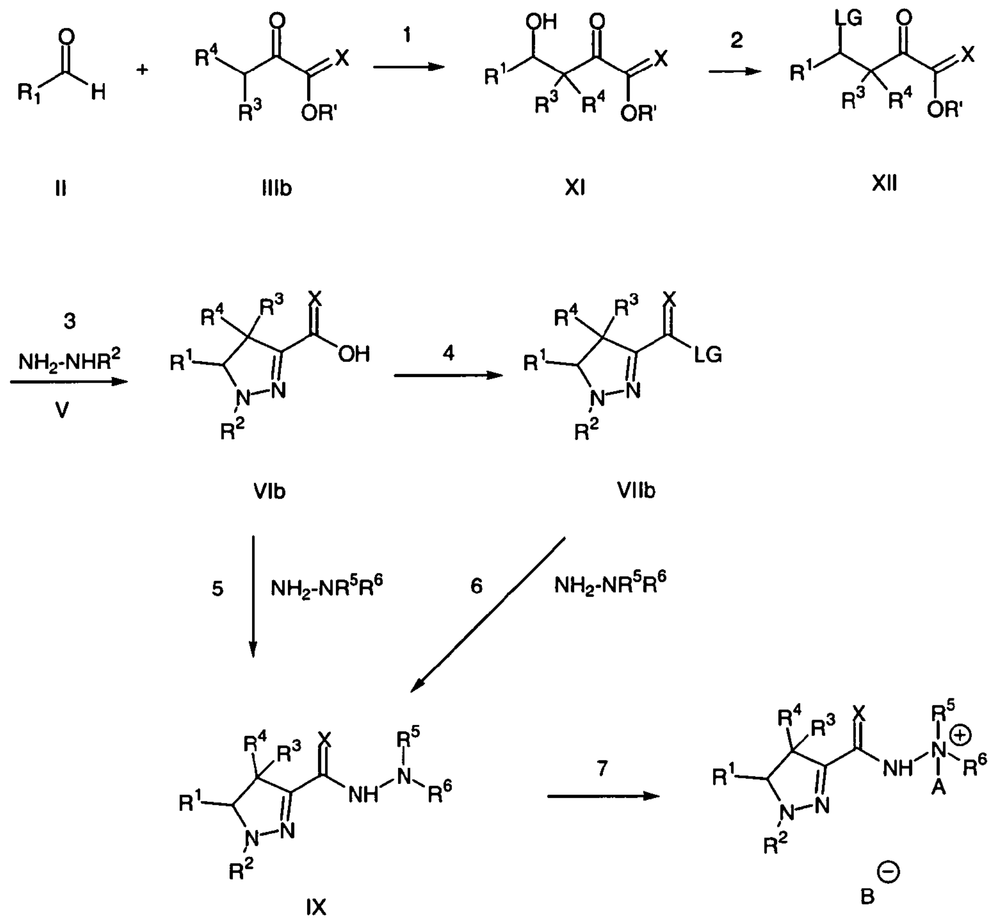


II IIIb XI XII

VIb VIIb

IX

Scheme II

In step 1 a compound of general formula IIIb is reacted with a compound of general formula II in a reaction medium , preferably in a protic reaction medium, more preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide or in the presence of lithium diisopropylamide in an aprotic solvent, preferably in tetrahydrofurane.

In step 2 a compound of general formula XI is transformed into a compound of general formula XII which contains a good living group LG, preferably a leaving group selected from the group consisting of mesyl and tosyl, using conventional methods known to those skilled in the art.

In step 3 a compound of general formula XII is reacted with a compound of general formula V in a reaction medium, preferably in a reaction medium selected from the group consisting of methanol, ethanol, isopropanol, n-butanol, dieethylether, tert-butyl-methylether, dioxane, tetrahydrofurane or mixtures of at least two of these afore mentioned reaction media. Also preferably, said reaction may be carried out in the presence of an acid, whereby the acid may be organic such as acetic acid and/or inorganic such as hydrochloric acid. Alternatively the reaction may also be carried out in the presence of a base such as piperidine, piperazine, sodium hydroxide, potassium hydroxide, sodium methoxide or sodium ethoxide or mixtures of at least two of these bases. Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

Step 4 can be carried out as described for step 3, scheme I; step 5 can be carried out as described for step 5, scheme I, step 6 can be carried out as described for step 4, scheme 1 and step 7 can be carried out as described for step 6, scheme I.

The afore mentioned reactions involving the synthesis of the 4,5-dihydro-pyrazole ring or the reaction of a compound comprising said ring are preferably carried out under an inert atmosphere, preferably under a nitrogen or argon atmosphere, to avoid oxidation of the ring-system.

During some synthetic reactions described above the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted pyrazoline compounds and/or the quaternary ammonium salts of substituted pyrazoline compounds of general formula I given above are obtained in form of a mixture of stereoisomers, particularly enantiomers or diastereomers, said mixtures may be separated by standard procedures known to those skilled in the art, e.g. chromatographic methods or crystallization with chiral reagents. It is also possible to obtain pure stereoisomers via stereoselective synthesis.

Solvates, preferably hydrates, of the quaternary ammonium salts of substituted pyrazoline compounds, of corresponding stereoisomers or of corresponding diastereomers thereof may also be obtained by standard procedures known to those skilled in the art.

The purification and isolation of the substituted pyrazoline compounds and/or of quaternary ammonium salts of substituted pyrazoline compounds, of a corresponding stereoisomer, or diastereomer or solvate or any intermediate thereof may, if required, be carried out by conventional methods known to those skilled in the art, e.g. chromatographic methods or recrystallization.

The quaternary ammonium salts of substituted pyrazoline compounds of general formula I given above may also act as prodrugs, i.e. they represent a drug precursor, which following administration to a patient releases a drug in vivo via some kind of chemical and/or physiological process (e.g., a prodrug on being brought to a physiological pH and/or through an enzyme action is converted to a desired drug form; see, e.g., R. B. Silverman, 1992, "The Organic Chemistry of Drug Design and Drug Action", Academic Press, Chp. 8). In particular, the compounds of general formula I give rise to a compound of general formula XIII,

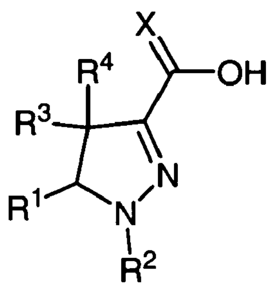

XIII

wherein $R^1$, $R^2$, $R^3$, $R^4$ and X have the meaning given above, upon administration to a patient.

Prodrugs can be used to alter the biodistribution (e.g., to allow compounds which would not typically enter the reactive site of the protease) or the pharmacokinetics for a particular compound. For example, a hydroxyl group, can be esterified, e.g., with a carboxylic acid group to yield an ester. When the ester is administered to a subject, the ester is cleaved, enzymatically or non-enzymatically, reductively or hydrolytically, to reveal the hydroxyl group.

The quaternary ammonium salts of substituted pyrazoline compounds of general formula I given above, their stereoisomers, their diastereomers and corresponding solvates are toxicologically acceptable and are therefore suitable as pharmaceutical active substances for the preparation of medicaments.

It has been found that quaternary ammonium salts of substituted pyrazoline compounds of general formula I given above, stereoisomers thereof, diastereomers thereof and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these quaternary ammonium salts of substituted pyrazoline compounds of general formula I given above show little or no development of tolerance during treatment, particularly with respect to food intake, i.e. if the treatment is interrupted for a given period of time and then continued afterwards, the inventively used quaternary ammonium salts of substituted pyrazoline compounds will again show the desired effect. After ending the treatment with the quaternary ammonium salts of substituted pyrazoline compounds of general formula I given above the positive influence on the body weight is found to continue.

Furthermore, these quaternary ammonium salts of substituted pyrazoline compounds show relatively weak Herg channel affinity, thus a low risk of prolongation of the QT-interval is to be expected for these compounds.

In summary, the inventively used quaternary ammonium salts of substituted pyrazoline compounds of general formula I given above are distinguished by a broad spectrum of beneficial effects, while at the same time showing relatively little undesired effects, i.e. effects which do not positively contribute to or even interfere with the well being of the patient.

Thus, an other aspect of the present invention relates to a medicament comprising at least quaternary ammonium salts of substituted pyrazoline compounds of general formula I given above, optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof, and optionally at least one physiologically acceptable auxiliary agent.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the quaternary ammonium salts of substituted pyrazoline compounds also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebella disorders, spinocerebella disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

Another aspect of the present invention is the use of at least one quaternary ammonium salt of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective quaternary ammonium salts of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of psychosis.

Also particularly preferred is the use of at least one of the respective quaternary ammonium salts of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the

preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the respective quaternary ammonium salts of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one quaternary ammonium salt of substituted pyrazoline compounds of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective quaternary ammonium salts of substituted pyrazoline compounds of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, , medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior

art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-subsituted phenyl-piperazine compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more quaternary ammonium salts of substituted pyrazoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000, even more preferably 1 to 150 milligrams of active substance to be administered during one or several intakes per day.

## Pharmacological Methods

### I. In-vitro determination of affinity to CB1/CB2-Receptors

The in-vitro determination of the affinity of the inventive quaternary ammonium salts of substituted pyrazoline compounds to $CB_1/CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is $[^3H]$-CP55940. The respective parts of the description is hereby incorporated by reference and forms part of the present disclosure.

### II. In-vivo bioassay system for determination of cannabinoid activity

### Mouse tetrad model

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202, 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

### Material and Methods

Male NMRI mice with a weight of 20-30 g (Harlan, Barcelona, Spain) are used in all of the following experiments.

Before testing in the behavioral procedures given below, mice are acclimatized to the experimental setting.

Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 ± 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\% \text{ MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

- **0**: no ataxia;
- **1**: doubful;
- **2**: obvious calmness and quiet;
- **3** pronounced ataxia;

prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is

calculated for each animal, whereby differences of ≥ -2 □C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:

more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

**III. In vivo testing for antiobesic activity**

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

**IV. In vivo testing for antidepressant activity**

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The present invention is illustrated below with the aid of examples. These illustrations are given solely by way of example and do not limit the general spirit of the present invention.

**Examples**

**Example 1: 1-[[5-(4-Chloro-phenyl)-1-(2,4-dichloro-phenyl)-4,5-dihydro-1H-pyrazole-3-carbonyl]-amino]-1-methyl-piperidinium iodide**

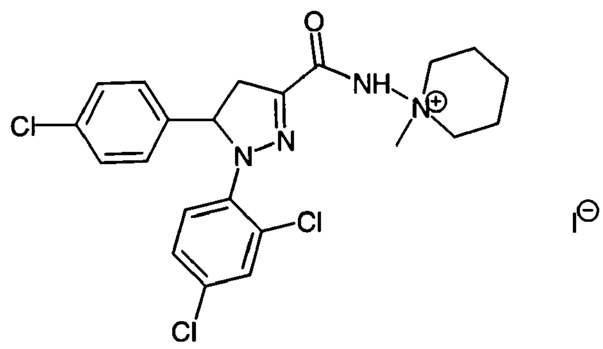

1-[[5-(4-Chloro-phenyl)-1-(2,4-dichloro-phenyl)-4,5-dihydro-1H-pyrazole-3-carboxylic acid piperidin-1-y-amide (1 mmol) was dissolved in chloroform (20 mL) and methyl iodide (3 mmol) was slowly added. The mixture was heated to reflux for 72 hours, excess methyl iodide was evaporated and the crude residue was crystallized in ethanol (5 mL) and a small amount of diethylether to afford the title compound as a yellow solid.

m.p. 174-176˚C

FTIR (KBr, $cm^{-1}$) 3448, 2948, 1675, 1480, 1248, 1116, 824

$^{1}$H NMR (400 MHz, METHANOL-$d_4$) δ ppm 1.66 (m, 1 H) 1.91 (m, 3 H) 2.05 (m, 2 H) 3.26 (dd, *J*=17.98. 6.64 Hz, 1 H) 3.67 (m, 2 H) 3.70 (s, 3 H) 3.74 (dd, *J*=17.98, 12.50 Hz, 1 H) 4.45 (d, *J*=12.90 Hz, 1 H) 4.57 (d, *J*=12.50 Hz, 1 H) 5.94 (dd, *J*=12.50, 6.64 Hz, 1 H) 7.22 (m, 5 H) 7.36 (d, *J*=2.34 Hz, 1 H) 7.41 (d, *J*=8.79 Hz, 1 H)

The following Examples 2 to 50 were prepared according to the process described for the synthesis of Example 1. Those skilled in the art are familiar with the starting materials that are needed to obtain said compounds.

| N° | STRUCTURE | Name | FTIR | m.p. °C |
|---|---|---|---|---|
| 1 | 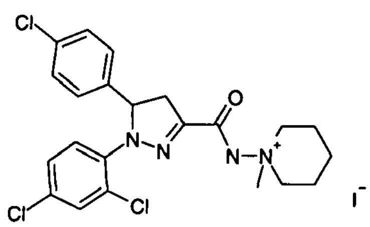 | 1-{[5-(4-Chloro-phenyl)-1-(2,4-dich loro-phenyl)-4,5-dihydro-1H-pyrazol e-3-carbonyl]-amino}-1-methyl-piper idinium; iodide | 3448, 2948, 1675, 1480, 1248, 1116, 824 $cm^{-1}$ | 174-176°C |
| 2 | 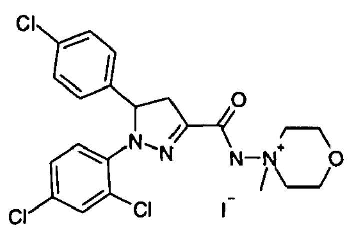 | 4-{[5-(4-Chloro-phenyl)-1-(2,4-dich loro-phenyl)-4,5-dihydro-1H-pyrazol e-3-carbonyl]-amino}-4-methyl-morph olin-4-ium; iodide | | |
| 3 | 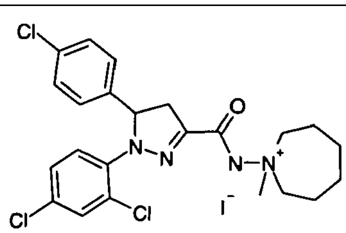 | 1-{[5-(4-Chloro-phenyl)-1-(2,4-dich loro-phenyl)-4,5-dihydro-1H-pyrazol e-3-carbonyl]-amino}-1-methyl-azepa nium; iodide | | |

(continued)

| N° | STRUCTURE | Name | FTIR | m.p. °C |
|---|---|---|---|---|
| 4 | 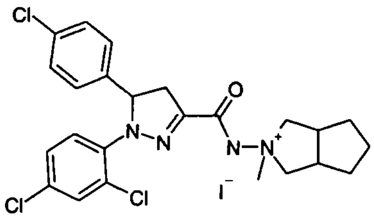 | 2-{[5-(4-Chloro-phenyl)-1-(2,4-dich loro-phenyl)-4,5-dihydro-1H-pyrazol e-3-carbonyl]-amino}-2-methyl-octah ydro-cyclopenta[c]pyrrolium; iodide | | |
| 5 | 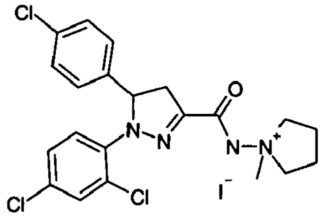 | 1-{[5-(4-Chloro-phenyl)-1-(2,4-dich loro-phenyl)-4,5-dihydro-1H-pyrazol e-3-carbonyl]-amino}-1-methyl-pyrro lidinium; iodide | | |
| 6 | 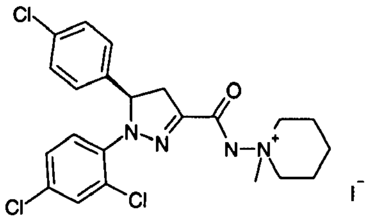 | (R)-1-{[5-(4-Chloro-phenyl)-1-(2,4-dich loro-phenyl)-4,5-dihydro-1H-pyrazol e-3-carbonyl]-amino}-1-methyl-piper idinium; iodide | 3448, 2944, 1696, 1477, 1245, 1230, 1104, 1086, 829 $cm^{-1}$ | 164-165 °C |
| 7 | 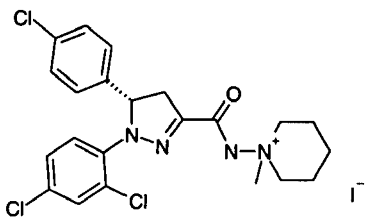 | (S)-1-{[5-(4-Chloro-phenyl)-1-(2,4-dich loro-phenyl)-4,5-dihydro-1H-pyrazol e-3-carbonyl]-amino}-1-methyl-piper idinium; iodide | 3448, 2944, 1696, 1477, 1245, 1230, 1104, 1086, 829 $cm^{-1}$ | 164-165 °C |
| 8 | 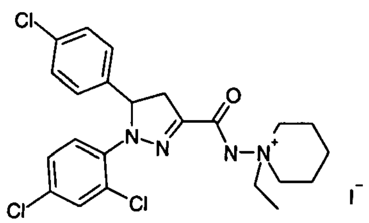 | 1-{[5-(4-Chloro-phenyl)-1-(2,4-dich loro-phenyl)-4,5-dihydro-1H-pyrazol e-3-carbonyl]-amino}-1-ethyl-piper idinium; iodide | | |
| 9 | 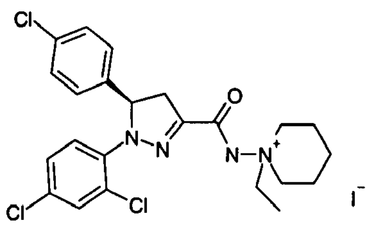 | (R)-1-{[5-(4-Chloro-phenyl)-1-(2,4-dich loro-phenyl)-4,5-dihydro-1H-pyrazol e-3-carbonyl]-amino}-1-ethyl-piperi dinium; iodide | | |
| 10 | 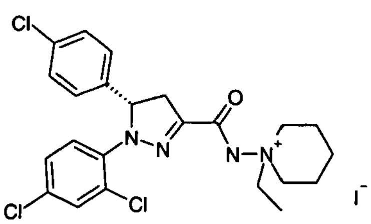 | (S)-1-{[5-(4-Chloro-phenyl)-1-(2,4-dich loro-phenyl)-4,5-dihydro-1H-pyrazol e-3-carbonyl]-amino}-1-ethyl-piperi dinium; iodide | | |

(continued)

| N° | STRUCTURE | Name | FTIR | m.p. °C |
|---|---|---|---|---|
| 11 | 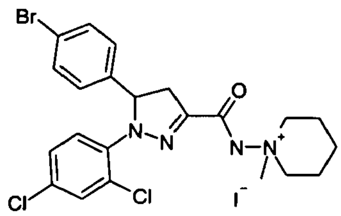 | 1-{[5-(4-Bromo-phenyl)-1-(2,4-dichl oro-phenyl)-4,5-dihydro-1H-pyrazole-3-carbonyl]-amino}-1-methyl-piperi dinium; iodide | | |
| 12 | 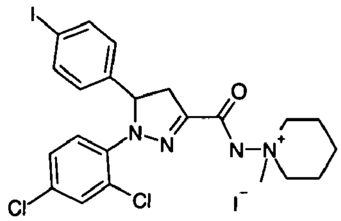 | 1-{[1-(2,4-Dichloro-phenyl)-5-(4-io do-phenyl)-4,5-dihydro-1H-pyrazole-3-carbonyl]-amino}-1-methyl-piperid inium; iodide | | |
| 13 | 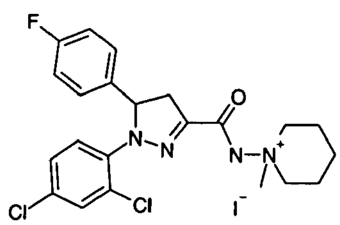 | 1-{[1-(2,4-Dichloro-phenyl)-5-(4-fl uoro-phenyl)-4,5-dihydro-1H-pyrazol e-3-carbonyl]-amino}-1-methyl-piper idinium; iodide | | |
| 14 | 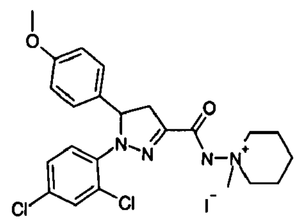 | 1-{[1-(2,4-Dichloro-phenyl)-5-(4-me thoxy-phenyl)-4,5-dihydro-1H-pyrazo le-3-carbonyl]-amino}-1-methyl-pipe ridinium; iodide | | |
| 15 | 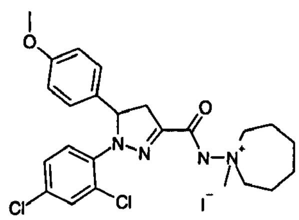 | 1-{[1-(2,4-Dichloro-phenyl)-5-(4-me thoxy-phenyl)-4,5-dihydro-1H-pyrazo le-3-carbonyl]-amino}-1-methyl-azep anium; iodide | | |
| 16 | 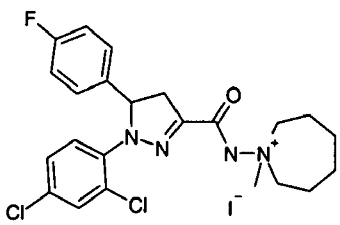 | 1-{[1-(2,4-Dichioro-phenyl)-5-(4-fl uoro-phenyl)-4,5-dihydro-1H-pyrazol e-3-carbonyl]-amino}-1-methyl-azepa nium; iodide | | |
| 17 | 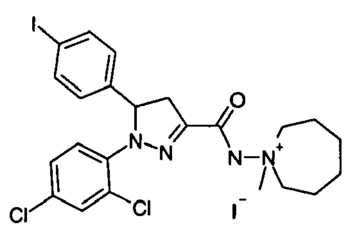 | 1-{[1-(2,4-Dichloro-phenyl)-5-(4-io do-phenyl)-4,5-dihydro-1H-pyrazole-3-carbonyl]-amino}-1-methyl-azepani um; iodide | | |

(continued)

| N° | STRUCTURE | Name | FTIR | m.p. °C |
|---|---|---|---|---|
| 18 | 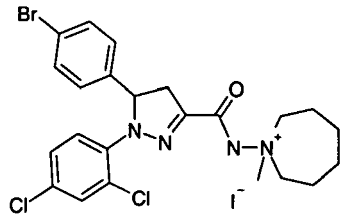 | 1-{[5-(4-Bromo-phenyl)-1-(2,4-dichl oro-phenyl)-4,5-dihydro-1H-pyrazole-3-carbonyl]-amino}-1-methyl-azepan ium; iodide | | |
| 19 | 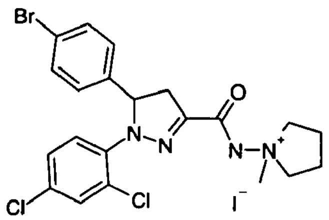 | 1-{[5-(4-Bromo-phenyl)-1-(2,4-dichl oro-phenyl)-4,5-dihydro-1H-pyrazole-3-carbonyl]-amino}-1-methyl-pyrrol idinium; iodide | | |
| 20 | 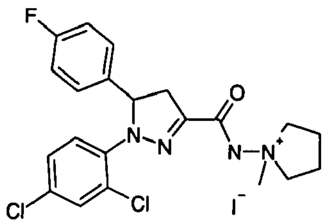 | 1-{[1-(2,4-Dichloro-phenyl)-5-(4-fl uoro-phenyl)-4,5-dihydro-1H-pyrazol e-3-carbonyl]-amino}-1-methyl-pyrro lidinium; iodide | | |
| 21 | 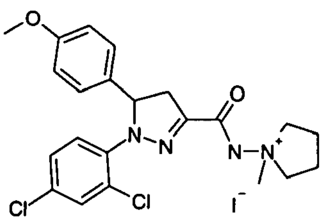 | 1-{[1-2,4-Dichloro-phenyl)-5-(4-methoxyphenyl)-4,5-dihydro-1H-pyrazole-3-carbonyl]-amino}-1-methyl-pyrrolidinium, iodide | | |
| 22 | 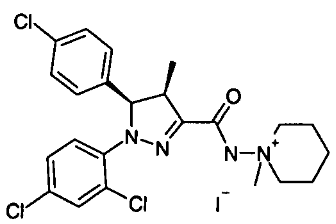 | cis-1-{[5-(4-Chloro-phenyl)-1-(2,4-dichloro-phenyl)-4-methyl-4,5-dihydro-1 H-pyrazole-3-carbonyl]-amino}-1-methyl piperidinium iodide | | |
| 23 | 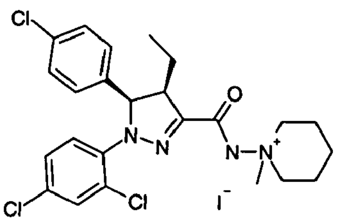 | cis-1-{[5-(4-Chloro-phenyl)-1-(2,4-dichloro-phenyl)-4-ethyl-4,5-dihydro-1 H-pyrazole-3-carbonyl]-amino}-1-methyl piperidinium iodide | | |
| 24 | 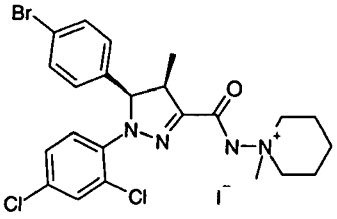 | cis-1-{[5-(4-Bromo-phenyl)-1-(2,4-dichloro-phenyl)-4-methyl-4,5-dihydro-1H-pyrazole-3-carbonyl]-amino}-1-methyl piperidinium iodide | | |

(continued)

| N° | STRUCTURE | Name | FTIR | m.p. °C |
|---|---|---|---|---|
| 25 | 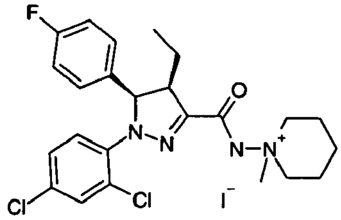 | cis-1-{[5-(4-Fluoro-phenyl)-1-(2,4-dichloro-phenyl)-4-ethyl-4,5-dihydro-1 H-pyrazole-3-carbonyl]-amino}-1-methyl piperidinium iodide | | |
| 26 | 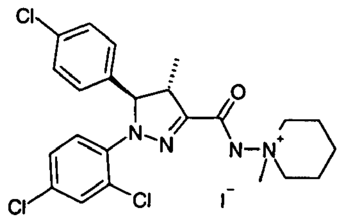 | trans-1-{[5-(4-Chloro-phenyl)-1-(2,4-dichloro-phenyl)-4-methyl-4,5-dihydro-1H-pyrazole-3-carbonyl]-amino}-1-methyl piperidinium iodide | | |
| 27 | 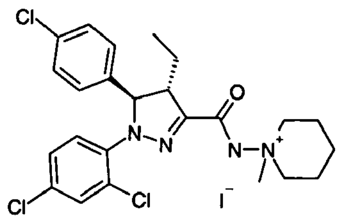 | trans-1-{[5-(4-Chloro-phenyl)-1-(2,4-dichloro-phenyl)-4-ethyl-4,5-dihydro-1 H-pyrazole-3-carbonyl]-amino}-1-methyl piperidinium iodide | | |
| 28 | 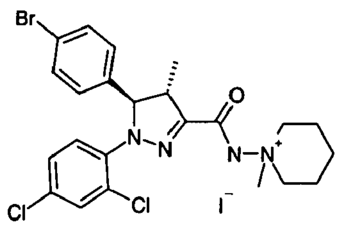 | trans-1-{[5-(4-Bromo-phenyl)-1-(2,4-dichloro-phenyl)-4-methyl-4,5-dihydro-1H-pyrazole-3-carbonyl]-amino}-1-methyl piperidinium iodide | | |
| 29 | 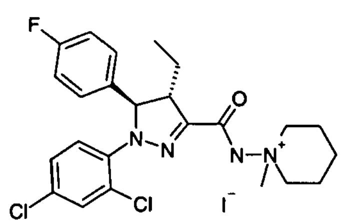 | trans-1-{[5-(4-Fluoro-phenyl)-1-(2,4-dichloro-phenyl)-4-ethyl-4,5-dihydro-1 H-pyrazole-3-carbonyl]-amino}-1-methyl piperidinium iodide | | |
| 30 | 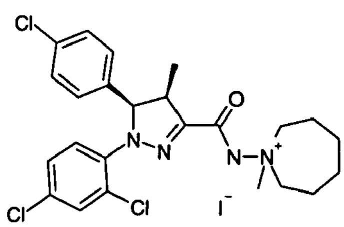 | cis-1-{[5-(4-Chloro-phenyl)-1-(2,4-dichloro-phenyl)-4-methyl-4,5-dihydro-1H-pyrazole-3-carbonyl]-amino}-1-methyl azepanium iodide | | |
| 31 | 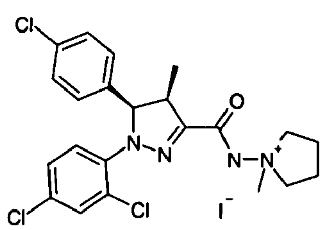 | cis-1-{[5-(4-Chloro-phenyl)-1-(2,4-dichloro-phenyl)-4-methyl-4,5-dihydro-1H-pyrazole-3-carbonyl]-amino}-1-methyl pyrrolidinium iodide | | |

(continued)

| N | STRUCTURE | Name | FTIR | m.p. C |
|---|---|---|---|---|
| 32 | 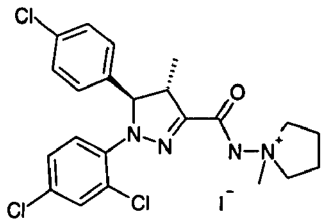 | trans-1-{[5-(4-Chloro-phenyl)-1-(2,4-dichloro-phenyl)-4-methyl-4,5-dihydro-1H-pyrazole-3-carbonyl]-amino}-1-methyl pyrrolidinium iodide | | |
| 33 | 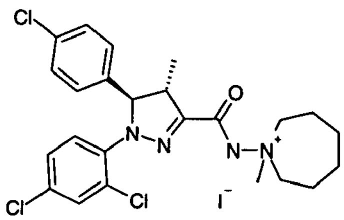 | trans-1-{[5-(4-Chloro-phenyl)-1-(2,4-dichloro-phenyl)-4-methyl-4,5-dihydro-1H-pyrazole-3-carbonyl]-amino}-1-methyl azepanium iodide | | |
| 34 | 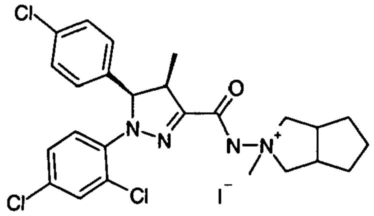 | cis-2-{[5-(4-Chloro-phenyl)-1-(2,4-dichloro-phenyl)-4-methyl-4,5-dihydro-1H-pyrazole-3-carbonyl]-amino}-2-methyl-octahydro-cyclopenta[c] pyrrolium iodide | | |
| 35 | 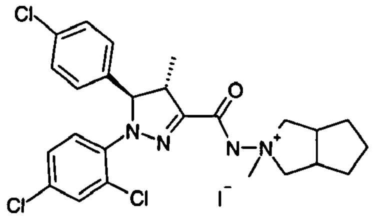 | trans-2-{[5-(4-Chloro-phenyl)-1-(2,4-dichloro-phenyl)-4-methyl-4,5-dihydro-1H-pyrazole-3-carbonyl]-amino}-2-methyl-octahydro-cyclopenta[c] pyrrolium iodide | | |
| 36 | 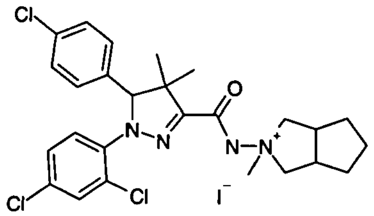 | 2-([5-(4-Chloro-phenyl)-1-(2,4-dichloro-phenyl)-4,4-dimethyl-4,5-dihydro-1H-pyrazole-3-carbonyl]-amino}-2-methyl-octahydro-cyclopenta[c] pyrrolium iodide | | |
| 37 | 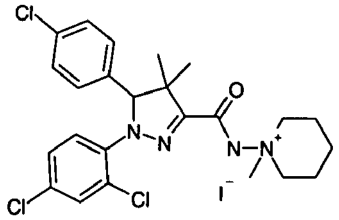 | 1-{[5-(4-Chloro-phenyl)-1-(2,4-dich loro-phenyl)-4,4-dimethyl-4,5-dihyd ro-1H-pyrazole-3-carbonyl]-amino}-1 -methyl-piperidinium; iodide | | |

(continued)

| N | STRUCTURE | Name | FTIR | m.p. C |
|---|---|---|---|---|
| 38 | 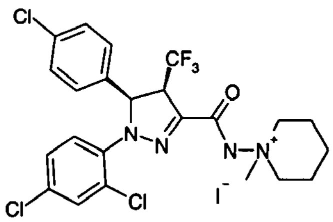 | cis-1-([5-(4-Chloro-phenyl)-1-(2,4-dichloro-phenyl)-4-trifluoromethyl-4,5-dihydro-1H-pyrazole-3-carbonyl]-amino}-1-methyl piperidinium iodide | | |
| 39 | 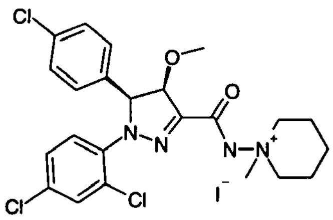 | cis-1-{[5-(4-Chloro-phenyl)-1-(2,4-dichloro-phenyl)-4-methoxy-4,5-dihydro-1H-pyrazole-3-carbonyl]-amino}-1-methyl piperidinium iodide | | |
| 40 | 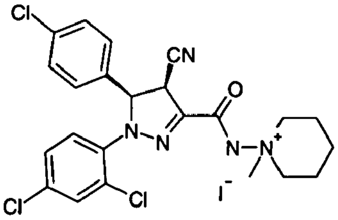 | cis-1-{[5-(4-Chloro-phenyl)-4-cyano-1-(2,4-dichloro-phenyl)-4,5-dihydro-1H-pyrazole-3-carbonyl]-amino}-1-methyl piperidinium iodide | | |
| 41 | 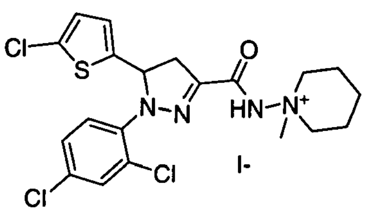 | 1-{[5-(5-Chloro-thiophen)-1-(2,4-dichloro-phenyl)-4,5-dihydro-1H-pyrazole-3-carbonyl]-amino}-1-methyl piperidinium iodide | | |
| 42 | 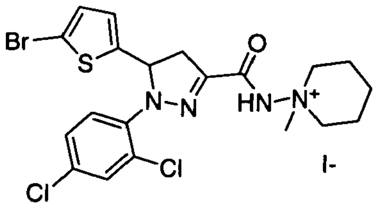 | 1-{[5-(5-Bromo-thiophen)-1-(2,4-dichloro-phenyl)-4,5-dihydro-1H-pyrazole-3-carbonyl]-amino}-1-methyl piperidinium iodide | | |
| 43 | 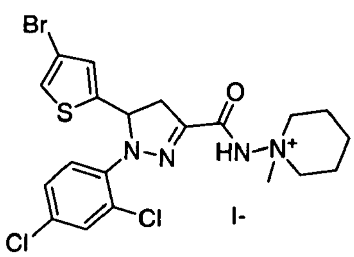 | 1-{[5-(4-Bromo-thiophen)-1-(2,4-dichloro-phenyl)-4,5-dihydro-1H-pyrazole-3-carbonyl]-amino}-1-methyl piperidinium iodide | | |

(continued)

| N° | STRUCTURE | Name | FTIR | m.p. °C |
|---|---|---|---|---|
| 44 | 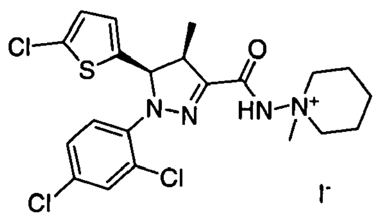 | cis-1-{[5-(5-Chloro-thiophen)-1-(2,4-dichloro-phenyl)-4-methyl-4,5-dihydro-1H-pyrazole-3-carbonyl]-amino}-1-methyl piperidinium iodide | | |
| 45 | 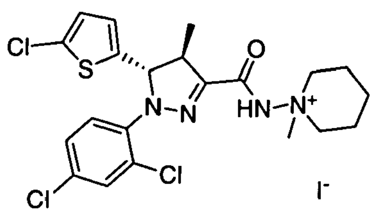 | trans-1-{[5-(5-Chloro-thiophen)-1-(2,4-dichloro-phenyl)-4-methyl-4,5-dihydro-1H-pyrazole-3-carbonyl]-amino}-1-methyl piperidinium iodide | | |
| 46 | 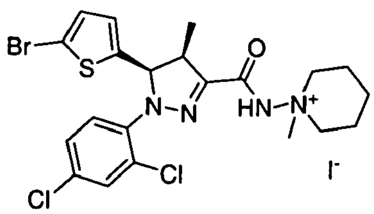 | cis-1-{[5-(5-Bromo-thiophen-2-yl)-1-(2, 4-dichloro-phenyl)-4-methyl-4,5-dihydro-1H-pyrazole-3-carbonyl]-amino}-1-methyl-piperidinium iodide | | |
| 47 | 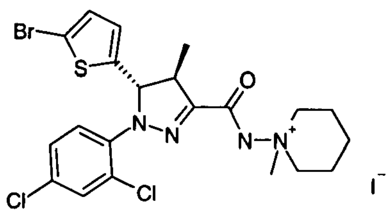 | trans- 1-{[5-(5-Bromo-thiophen-2-yl)-1-(2, 4-dichloro-phenyl)-4-methyl-4,5-dihydro-1H-pyrazole-3-carbonyl]-amino}-1-methyl-piperidinium iodide | | |
| 48 | 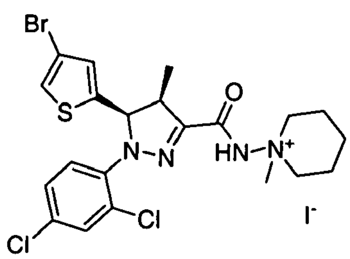 | cis-1-{[5-(5-Bromo-thiophen)-1-(2,4-dichloro-phenyl)-4-methyl-4,5-dihydro-1H-pyrazole-3-carbonyl]-amino}-1-methyl piperidinium iodide | | |
| 49 | 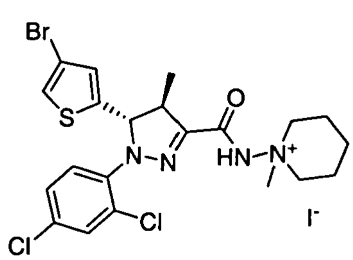 | trans-1-{[5-(5-Bromo-thiophen)-1-(2,4-dichloro-phenyl)-4-methyl-4,5-dihydro-1H-pyrazole-3-carbonyl]-amino}-1-methyl piperidinium iodide | | |

(continued)

| N | STRUCTURE | Name | FTIR | m.p. C |
|---|---|---|---|---|
| 50 | 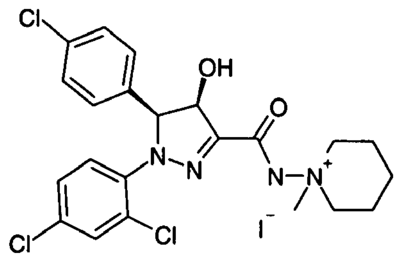 | cis-1-{[5-(4-Chloro-phenyl)-1-(2,4-dichloro-phenyl)-4-hydroxy-4,5-dihydro-1H-pyrazole-3-carbonyl]-amino}-1-methyl piperidinium iodide | | |

## Claims

**1.** A quaternary ammonium salt of general formula I,

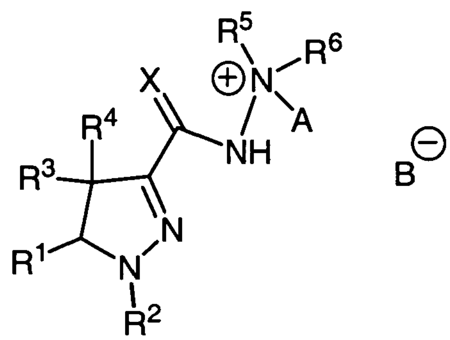

I

wherein

X isOorS;
A represents a hydrogen atom or an unsubstituted or at least mono-substituted alkyl radical;
$R^1$ and $R^2$, independently of one another, in each case represent an unsubstituted or at least mono-substituted aryl or heteroaryl radical, which may be condensed with an unsubstituted or at least mono-substituted mono- or polycyclic ring system;
or a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;
$R^3$ and $R^4$, independently of one another, in each case represent H; F; Cl; Br; I; -CN; -$NO_2$; -NC; -OH; -$NH_2$; -SH; -C(=O)-H;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;
a —O-$R^7$ moiety; a —S-$R^8$ moiety; a —NH-$R^9$ moiety or a —$NR^{10}R^{11}$ moiety;

$R^5$ and $R^6$, independently of one another, in each case represent a hydrogen atom; a saturated or unsaturated, unsubstituted or at least mono-substituted aliphatic radical;

or

$R^5$ and $R^6$ together with the bridging nitrogen form an unsubstituted or at least mono-substituted, saturated or unsaturated heterocyclic ring which may contain at least one further heteroatom as a ring member and/or which may be condensed with one or two optionally at least mono-substituted mono- or polycyclic ring systems;
$R^7$, $R^8$, $R^9$, $R^{10}$ and $R^{11}$, independently of one another, in each case represent a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxoglutarate, formate, acetate, propionate, lactate, gluconate, unsubstituted or at least mono-substituted benzoate or naphthoate, pyruvate, ascorbate,

$$R^{12}\text{-}SO_3^{\ominus} \quad \text{and} \quad R^{13}\text{-NH-}SO_3^{\ominus};$$

glycolate, nicotinate, phenylacetate,
and

$R^{12}$ and $R^{13}$, independently of one another, in each case, represent a saturated or unsaturated, unsubstituted or at least mono-substituted aliphatic radical;
an unsubstituted or at least mono-substituted aryl or heteroaryl radical which may be bonded via an unsubstituted or at least mono-substituted alkylene group
or a saturated or unsaturated, unsubstituted or at least mono-substituted, optionally at least one heteroatom as a ring member containing cycloaliphatic radical, which may be bridged by at least one unsubstituted or at least mono-substituted alkylene group and/or may be bonded via an unsubstituted or at least mono-substituted alkylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**2.** A salt according to claim 1, **characterized in that**

X isOorS;
A represents a hydrogen atom or an unsubstituted or at least mono-substituted $C_{1-10}$ alkyl radical;
$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or

polycyclic ring system and/or may be bridged by at least one unsubstituted or at least mono-substituted $C_{1-5}$ alkylene group;

$R^3$ and $R^4$, independently of one another, in each case represent H; F; Cl; Br; I; -CN; $-NO_2$; -NC; -OH; $-NH_2$; -SH; -C(=O)-H;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

a —O-$R^7$ moiety; a —S-$R^8$ moiety; a —NH-$R^9$ moiety or a —$NR^{10}R^{11}$ moiety;

$R^5$ and $R^6$, independently of one another, in each case represent a hydrogen atom;

an unsubstituted or at least mono-substituted $C_{1-16}$ alkyl radical, $C_{2-16}$ alkenyl radical or $C_{2-16}$ alkinyl radical;

or

$R^5$ and $R^6$ together with the bridging nitrogen form an unsubstituted or at least mono-substituted, saturated or unsaturated 4- to 10-membered heterocyclic ring which may optionally contain 1, 2 or 3 additional heteroatom (s) selected from the group consisting of sulfur, nitrogen and oxygen as ring member(s) and/or which may be condensed with one or two optionally at least mono-substituted mono- or polycyclic ring systems;

$R^7$, $R^8$, $R^9$, $R^{10}$ and $R^{11}$, independently of one another, in each case represent

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with

an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxoglutarate, formate, acetate, propionate, lactate, gluconate, unsubstituted or at least mono-substituted benzoate or naphthoate, pyruvate, ascorbate,

$$R^{12}\text{-}SO_3^{\ominus} \quad \text{and} \quad R^{13}\text{-NH-}SO_3^{\ominus};$$

glycolate, nicotinate, phenylacetate,
and

$R^{12}$ and $R^{13}$, independently of one another, in each case, represent
an unsubstituted or at least mono-substituted $C_{1-16}$alkyl radical, $C_{2-16}$ alkenyl radical or $C_{2-16}$ alkinyl radical;
an unsubstituted or at least mono-substituted 6- or 10-membered aryl radical, which may be bonded via an unsubstituted or at least mono-substituted $C_{1-5}$ alkylene group;
an unsubstituted or at least mono-substituted 5- to 14-membered heteroaryl radical, which may be bonded via an unsubstituted or at least mono-substituted $C_{1-5}$ alkylene group, $C_{2-5}$ alkenylene group or $C_{2-5}$ alkinylene group;
an unsubstituted or at least mono-substituted $C_{3-16}$ cycloalkyl radical or $C_{4-16}$ cycloalkenyl radical, which in each case may be bonded via an unsubstituted or at least mono-substituted $C_{1-5}$ alkylene group and/or may be bridged by at least one unsubstituted or at least mono-substituted $C_{1-5}$ alkylene group;
or an unsubstituted or at least mono-substituted $C_{4-16}$ heterocycloalkyl radical or $C_{5-16}$ heterocycloalkenyl radical, which in each case may be bonded via an unsubstituted or at least mono-substituted $C_{1-5}$ alkylene group and/or may be bridged by at least one unsubstituted or at least mono-substituted $C_{1-5}$ alkylene group;

whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member (s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**3.** A salt according to claim 1 or 2, **characterized in that** A represents a monovalent oxygen anion ($O^-$) or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and —$NO_2$.

**4.** A salt according to one or more of claims 1 to 3, **characterized in that** $R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo [b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of —$CF_3$, $-CH_2-Cl$, methyl, ethyl, n-propyl, isopropyl, n-butyl, isobutyl, 2-butyl, tert-butyl, n-pentyl, 2-pentyl, n-hexyl, $-0-CH_3$, $-O-C_2H_5$, $-O-CH_2-CH_2-CH_3$, $-O-CH(CH_3)_2$, $-O-CH_2-CH_2-CH_2-CH_3$, $-0-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, - $NO_2$, -CHO, $-C(=O)-CH_3$, $-C(=O)-C_2H_5$, $-C(=O)-C(CH_3)_3$, $-CF_2H$, $-CFH_2$, - $C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, - $N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, decahydronaphthyl, octahydro-cyclopenta[c]pyrrolyl, decahydroquinolinyl and dodecahydro-carbazolyl,
which may optionally be substituted with 1, 2, 3, 4 or 5 substituent(s) independently selected from the group consisting of oxo (=O), thioxo (=S), - $CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, - $O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, - $SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, - $C(=O)-NH-C_3H_7$, $-C(=O)-N(CH_3)_2$, $-C(=O)-N(C_2H_5)_2$, $-S(=O)-CH_3$, -S $(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$.

**5.** A salt according to one or more of claims 1 to 4, **characterized in that** $R^3$ and $R^4$, independently of one another, in each case represent H; F; Cl; Br; I; -CN; - $NO_2$; -NC; -OH; $-NH_2$; -SH; -C(=O)-H;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, -0-CH $(CH_3)_2$, $-O-CH_2-CH_2-CH_2-CH_3$, $-O-C(CH_3)_3$, $-NH_2$, $-NH-CH_3$, $-NH-C_2H_5$, - $N(CH_3)_2$,$-N(C_2H_5)_2$, -CN and $-NO_2$;
a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, - $(CH_2)-(CH_2)-(CH_2)-$ or —CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, -O- $C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, - $C(=O)-C(CH_3)_3$, $-CF_2H$, $-CFH_2$, $-C(=O)-NH_2$, $-C(=O)-NH-CH_3$, -C $(=O)-NH-C_2H_5$,- $C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, -S $(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may be bonded via a — $(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, -S- $C(CH_3)_3$, F, Cl, Br, I, -CN, $-OCF_3$, $-SCF_3$, $-SCF_2H$, $-SCFH_2$,-OH, -SH, $-NO_2$, -CHO, $-C(=O)-CH_3$, $-C(=O)-C_2H_5$, -C $(=O)-C(CH_3)_3$, $-CF_2H$, - $CFH_2$, $-C(=O)-NH_2$, $-C(=O)-NH-CH_3$, $-C(=O)-NH-C_2H_5$, $-C(=O)-NH-C_3H_7$, - $C(=O)-N(CH_3)_2$, $-C(=O)-N(C_2H_5)_2$, $-S(=O)-CH_3$, $-S(=O)-C_2H_5$, $-S(=O)-C_3H_7$, - $S(=O)_2-CH_3$, $-S(=O)_2-C_2H_5$, $-S(=O)_2-C_3H_7$, $-NH_2$, -NH- $CH_3$, $-NH-C_2H_5$, $-N(CH_3)_2$ and $-N(C_2H_5)_2$;
a $-O-R^7$ moiety; a $-S-R^8$ moiety, a $-NH-R^9$ moiety or a $-NR^{10}R^{11}$ moiety.

**6.** A salt according to one or more of claims 1 to 5, **characterized in that** $R^5$ and $R^6$, independently of one another, in each case represent a hydrogen atom;
or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-0-CH_3$, $-O-C_2H_5$, $-O-CH_2-CH_2-CH_3$, $-O-CH(CH_3)_2$, $-O-CH_2-CH_2-CH_2-CH_3$, $-0-C(CH_3)_3$,- $S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $—NO_2$.

7. A salt according to one or more of claims 1 to 5, **characterized in that** $R^5$ and $R^6$ together with the bridging nitrogen atom form a moiety selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (2,5)-dihydro-1 H-pyrrolyl, (2,3)-dihydro-1 H-pyrrolyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, indolinyl, isoindolinyl, (1,2,3,4)-tetrahydroquinolinyl, (1,2,3,4)-tetrahydroisoquinolinyl, octahydrocyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl and (1,2,3,4)-tetrahydroquinoxazlinyl, which may optionally be substituted with 1, 2, 3, 4 or 5 substituent(s) independently selected from the group consisting of oxo (=O), thioxo (=S), - $CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, - O-$CH_2$-$CH_2$-$CH_2$-$CH_3$, -O-C($CH_3$)$_3$, -O-$CH_2$-O-$CH_3$, -O-$CH_2$-$CH_2$-O-$CH_3$, -0-$CH_2$-O-$C_2H_5$, -C($OCH_3$)($C_2H_5$)$_2$, -C($OCH_3$)($CH_3$)$_2$, -$CH_2$-O-$CH_3$, -$CH_2$-O-$C_2H_5$, - S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, - CHO, -C(=O)-$CH_3$, -C(=O)-$C_2H_5$, -C(=O)-C($CH_3$)$_3$, -C(=O)-OH, -C(=O)-O-$CH_3$, -C(=O)-O-$C_2H_5$, -C(=O)-O-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, - S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$, -N($C_2H_5$)$_2$, cyclopentyl, cyclohexyl, pyrrolidinyl and piperidinyl.

8. A salt according to one or more of claims 1 to 7, **characterized in that**
$R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -$NH_2$, -NH-$CH_3$, -NH-$C_2H_5$, -N($CH_3$)$_2$, —N($C_2H_5$)$_2$, -CN and -$NO_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a -($CH_2$)-, -($CH_2$)-($CH_2$)-, -($CH_2$)-($CH_2$)-($CH_2$)- or — CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -0-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, - C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and -N($C_2H_5$)$_2$;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a -($CH_2$)-, -($CH_2$)-($CH_2$)-, - ($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -0-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, - C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and —N($C_2H_5$)$_2$.

9. A salt according to one or more of claims 1 to 8, **characterized in that** B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, - OH, -O-$CH_3$ and -O-$C_2H_5$, pyruvate, ascorbate, glycolate, nicotinate, phenylacetate,

$$R^{12}\text{-}SO_3^{\ominus} \quad \text{and} \quad R^{13}\text{-}NH\text{-}SO_3^{\ominus}.$$

**10.** A salt according to one or more of claims 1 to 9, **characterized in that** $R^{12}$ and $R^{13}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl, n-tetradecyl, vinyl, n-propenyl, n-butenyl, n-pentenyl, n-hexenyl, ethinyl, propinyl, n-butinyl, n-pentinyl and n-hexinyl; which may optionally be substituted with 1, 2, 3, 4, 5, 6, 7, 8 or 9 substituent(s) independently selected from the group consisting of F, Cl, Br, $-NH_2$, $-NH-CH_3$, $-NH-C_2H_5$, $-N(CH_3)_2$ and $-N(C_2H_5)_2$;
a radical selected from the group consisting of phenyl, naphthyl, pyridinyl, benzimidazolyl, isoquinolinyl and pyrazolyl, which may be bonded via a $-(CH_2)-$, $-(CH_2)-(CH_2)-$ or $-(CH_2)-(CH_2)-(CH_2)-$group and/or may be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-SO_3H$, $-NH_2$, $-NH-CH_3$, $-NH-C_2H_5$, $-N(CH_3)_2$, $-N(C_2H_5)_2$ and phenyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a $-(CH_2)-$, $-(CH_2)-(CH_2)-$, $-(CH_2)-(CH_2)-(CH_2)-$group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-0-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-C(=O)-OH$, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$.

**11.** A salt according to one or more of claims 1 to 10, **characterized in that**
X is O or S:
A represents a monovalent oxygen anion ($O^-$) or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $-NO_2$;
$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of $-CF_3$, $-CH_2-Cl$, methyl, ethyl, n-propyl, isopropyl, n-butyl, isobutyl, 2-butyl, tert-butyl, n-pentyl, 2-pentyl, n-hexyl, $-0-CH_3$, $-O-C_2H_5$, $-O-CH_2-CH_2-CH_3$, $-O-CH(CH_3)_2$, $-O-CH_2-CH_2-CH_2-CH_3$, $-0-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, decahydronaphthyl, octahydro-cyclopenta[c]pyrrolyl, decahydroquinolinyl and dodecahydro-carbazolyl,

which may optionally be substituted with 1, 2, 3, 4 or 5 substituent(s) independently selected from the group consisting of oxo (=O), thioxo (=S), - $CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, - $O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, - $SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, - $C(=O)-NH-C_3H_7$, $-C(=O)-N(CH_3)_2$, $-C(=O)-N(C_2H_5)_2$, $-S(=O)-CH_3$, -S $(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

$R^3$ and $R^4$, independently of one another, in each case represent H; F; Cl; Br; I; -CN; $-NO_2$; -NC; -OH; $-NH_2$; -SH; -C(=O)-H;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, -0-CH $(CH_3)_2$, $-O-CH_2-CH_2-CH_2-CH_3$, $-O-C(CH_3)_3$, $-NH_2$, $-NH-CH_3$, $-NH-C_2H_5$, - $N(CH_3)_2$,$-N(C_2H_5)_2$, -CN and $-NO_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, - $(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, - $C(=O)-C(CH_3)_3$, $-CF_2H$, $-CFH_2$, $-C(=O)-NH_2$, $-C(=O)-NH-CH_3$, -C $(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, -S $(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may be bonded via a — $(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, -S-$C(CH_3)_3$, F, Cl, Br, I, -CN, $-OCF_3$, $-SCF_3$, $-SCF_2H$, $-SCFH_2$, - OH, -SH, $-NO_2$, -CHO, $-C(=O)-CH_3$, $-C(=O)-C_2H_5$, -C $(=O)-C(CH_3)_3$, $-CF_2H$, - $CFH_2$, $-C(=O)-NH_2$, $-C(=O)-NH-CH_3$, $-C(=O)-NH-C_2H_5$, $-C(=O)-NH-C_3H_7$, - $C(=O)-N(CH_3)_2$, $-C(=O)-N(C_2H_5)_2$, $-S(=O)-CH_3$, $-S(=O)-C_2H_5$, $-S(=O)-C_3H_7$, - $S(=O)_2-CH_3$, $-S(=O)_2-C_2H_5$, $-S(=O)_2-C_3H_7$, $-NH_2$, -NH-$CH_3$, $-NH-C_2H_5$, $-N(CH_3)_2$ and $—N(C_2H_5)_2$;

a $—O-R^7$ moiety; a $—S-R^8$ moiety, a $—NH-R^9$ moiety or a $—NR^{10}R^{11}$ moiety;

$R^5$ and $R^6$, independently of one another, in each case represent a hydrogen atom;

or a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4 or 5 substituent(s) independently selected from the group consisting of $-0-CH_3$, $-O-C_2H_5$, $-O-CH_2-CH_2-CH_3$, $-O-CH(CH_3)_2$, $-O-CH_2-CH_2-CH_2-CH_3$, $-0-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -CN and $—NO_2$;

or

$R^5$ and $R^6$ together with the bridging nitrogen atom form a moiety selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (2,5)-dihydro-1 H-pyrrolyl, (2,3)-dihydro-1 H-pyrrolyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, indolinyl, isoindolinyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl and (1,2,3,4)-tetrahydroquinoxazlinyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

$R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a $-(CH_2)-$, $-(CH_2)-(CH_2)-$, $-(CH_2)-(CH_2)-(CH_2)-$ or — CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, -OH, $-O-CH_3$ and $-O-C_2H_5$, pyruvate, ascorbate, glycolate, nicotinate, phenylacetate,

$$R^{12}\text{-}SO_3^{\ominus} \quad \text{and} \quad R^{13}\text{-NH-}SO_3^{\ominus};$$

and
$R^{12}$ and $R^{13}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl, n-tetradecyl, vinyl, n-propenyl, n-butenyl, n-pentenyl, n-hexenyl, ethinyl, propinyl, n-butinyl, n-pentinyl and n-hexinyl; which may optionally be substituted with 1, 2, 3, 4, 5, 6, 7, 8 or 9 substituent(s) independently selected from the group consisting of F, Cl, Br, $-NH_2$, $-NH-CH_3$, $-NH-C_2H_5$, $-N(CH_3)_2$ and $-N(C_2H_5)_2$;
a radical selected from the group consisting of phenyl, naphthyl, pyridinyl, benzimidazolyl, isoquinolinyl and pyrazolyl, which may be bonded via a — $(CH_2)$-, $-(CH_2)-(CH_2)-$ or $-(CH_2)-(CH_2)-(CH_2)$-group and/or may be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, - $C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-SO_3H$, $-NH_2$, - $NH-CH_3$, $-NH-C_2H_5$, $-N(CH_3)_2$, —$N(C_2H_5)_2$ and phenyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a —$(CH_2)$-, $-(CH_2)-(CH_2)$-, $-(CH_2)-(CH_2)-(CH_2)$-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, - $C(OCH_3)(C_2H_5)_2$, $-C(OCH_3)(CH_3)_2$, $-CH_2-O-CH_3$, $-CH_2-O-C_2H_5$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-C(=O)-OH$, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, - $S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, - $NH-CH_3$, $-NH-C_2H_5$, $-N(CH_3)_2$ and —$N(C_2H_5)_2$.
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**12.** A salt according to one or more of claims 1 to 11, **characterized in that**
X is O or S;
A represents a monovalent oxygen anion($O^-$) or a radical selected from the group consisting of methyl, ethyl, n-propyl, isopropyl, n-butyl, isobutyl, sec-butyl and tert-butyl;
$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of —$CF_3$, $-CH_2-Cl$, $-O-CH_3$, $-O-C_2H_5$, $-0-CH_2-CH_2-CH_3$, $-O-CH(CH_3)_2$, $-O-CH_2-CH_2-CH_2-CH_3$, $-O-C(CH_3)_3$, methyl, ethyl, n-propyl, isopropyl, n-butyl, isobutyl, 2-butyl, tert-butyl, n-pentyl, 2-pentyl, n-hexyl, F, Cl, Br, I, -CN, $-OCF_3$, $-SCF_3$, $-SCF_2H$, $-SCFH_2$, -OH, -SH, $-NO_2$, - CHO, $-CF_2H$, $-CFH_2$, phenoxy and thiophenyl, whereby the thiophenyl radical can be substituted with 1, 2 or 3 substituents independently selected from the group consisting of F, Cl, Br, methyl, ethyl and n-propyl;
$R^3$ represents H; F; Cl; Br; I; -CN; $-NO_2$; -NC; -OH; $-NH_2$; -SH; -C(=O)-H;
a radical selected from the group consisting of methyl, $-CF_3$, $-CH_2F$, $-CF_2H$, - $C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a radical selected from the group consisting of phenyl, naphthyl, pyridinyl, furyl (furanyl), thienyl (thiophenyl) and pyrrolyl, which may be bonded via a —$(CH_2)$-group and/or may optionally be substituted with 1, 2, 3, 4 or 5 substituent (s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, $-0-CH_3$, $-O-C_2H_5$, F, Cl and Br;
a —$O-R^7$ moiety; a —$S-R^8$ moiety, a —$NH-R^9$ moiety or a —$NR^{10}R^{11}$ moiety;

$R^4$ represents H or an alkyl radical selected from the group consisting of methyl, ethyl, n-propyl, isopropyl and n-butyl; $R^7$, $R^8$, $R^9$, $R^{10}$ and $R^{11}$, independently of another, in each case represent a radical selected from the group consisting of methyl, ethyl, n-propyl, isopropyl, n-butyl, isobutyl, sec-butyl, tert-butyl and n-pentyl;
$R^5$ and $R^6$ together with the bridging nitrogen atom form a moiety selected from the group consisting of

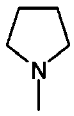 , 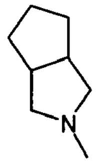 , 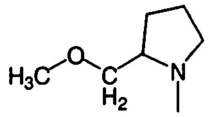 , 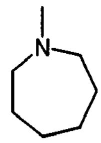 , 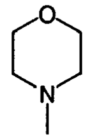 , 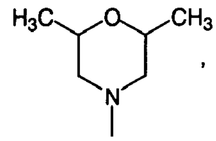 ,

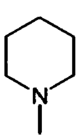 , 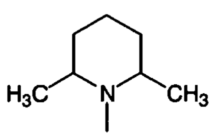 , 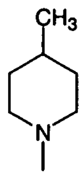 , 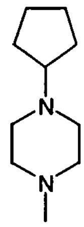 , 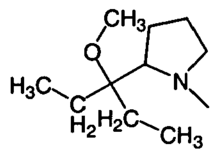 , 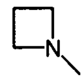 ,

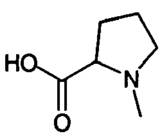 , 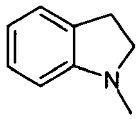 , 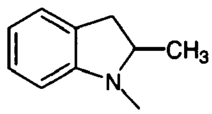 , 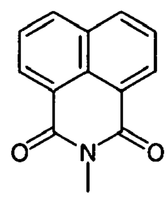 , 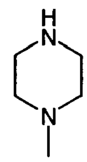 ,

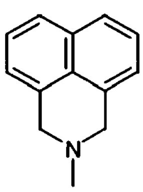 , 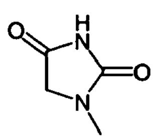 , 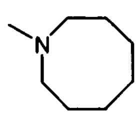 , 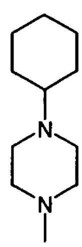 , 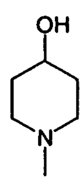 , 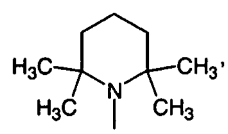 ,

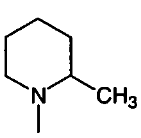 , 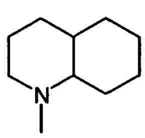 , 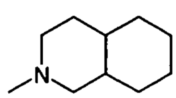 , 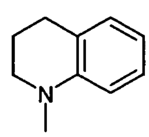 , 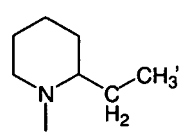 ,

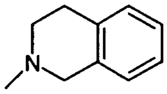 , 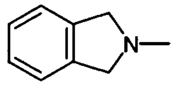 , 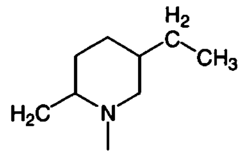 and 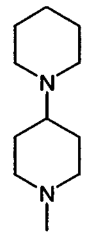 ;

and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, fumarate, citrate, glutarate, succinate, maleate, tartrate, phosphate, 2-oxo-glutarate, formate, acetate, propionate, lactate, gluconate, benzoate or naphthoate which may be substituted with 1, 2, 3, 4 or 5 substituents independently selected from the group consisting of F, Cl, Br, -OH, -O-$CH_3$ and -O-$C_2H_5$ , pyruvate, ascorbate, glycolate, nicotinate, phenylacetate,

$$R^{12}\text{-}SO_3^{\ominus} \quad \text{and} \quad R^{13}\text{-}NH\text{-}SO_3^{\ominus};$$

and
$R^{12}$ and $R^{13}$, independently of one another, in each case represent a radical selected from the group consisting of -$CF_3$, -$C_2F_5$, methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, n-nonyl, n-decyl, n-undecyl, n-dodecyl, n-tridecyl and n-tetradecyl;
a radical selected from the group consisting of phenyl, pyridinyl, pyrazolyl, benzimidazolyl, isoquinolinyl and naphthyl, which may be substituted with 1, 2, 3, 4 or 5 substituent(s) independently selected from the group consisting of F, Cl, Br, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, - O-$CH_2$-$CH_2$-$CH_2$-$CH_3$, -O-$C(CH_3)_3$, -OH, -$NO_2$, -$NH_2$, phenyl and -$SO_3H$;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl and bicyclo[2.2.1]heptyl, which may be bonded via a —($CH_2$)-, -($CH_2$)-($CH_2$)-, -($CH_2$)-($CH_2$)-($CH_2$)-group;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**13.** A salt according to one or more of claims 1 to 12, **characterized in that**

X is O;
A represents a monovalent oxygen anion ($O^-$) or a radical selected from the group consisting of methyl, ethyl and n-propyl;
$R^1$ represents a radical selected from the group consisting of phenyl and thienyl (thiophenyl), which may optionally be substituted with 1, 2 or 3 substituent(s) independently selected from the group consisting of F, Cl, Br, I, -O-$CH_3$ and —O-$C_2H_5$;
$R^2$ represents a phenyl radical, which may be substituted with 1, 2 or 3 substituent(s) independently selected from the group consisting of F, Cl, Br and I;
$R^3$ represents H; F; Cl; Br; I; -OH, -CN; -C(=O)-H;
a radical selected from the group consisting of methyl, -$CF_3$, -$CH_2F$,-$CF_2H$, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl, -$CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;
a benzyl radical or a —O-$R^7$ moiety;
$R^4$ represents H or a radical selected from the group consisting of methyl, ethyl, n-propyl, iso-propyl and n-butyl;
$R^5$ and $R^6$ together with the bridging nitrogen atom form a moiety selected from the group consisting of

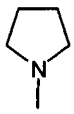, 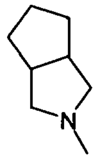, 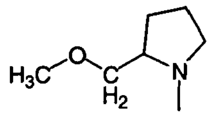, 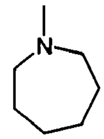, 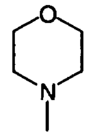, 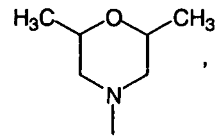,

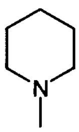, 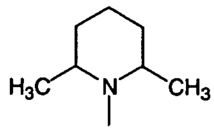, 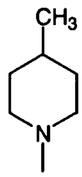, 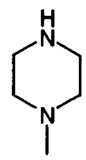, 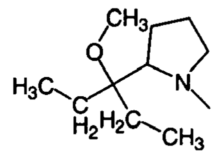,

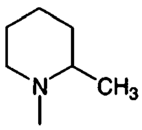, 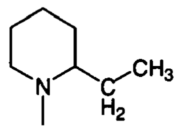, 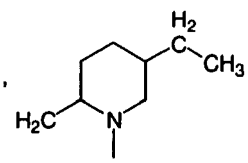, 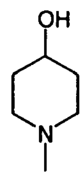 and 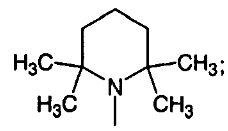;

$R^7$ represents a radical selected from the group consisting of methyl, ethyl, n-propyl, isopropyl, n-butyl, isobutyl, sec-butyl, tert-butyl and n-pentyl;
and in case A is not a monovalent oxygen anion, B represents an anion selected from the group consisting of chloride, bromide, iodide, fluoride, hydrogensulfate, nitrate, dihydrogenphosphate, thiocyanate, cyanate, acrylate, methanesulfonate, ethanesulfonate, toluenesulfonate, benzenesulfonate, (2,5)-dihydroxy-benzenesulfonate, naphthalene-2-sulfonate, 5-sulfonapthalene-1-sulfonate, cyclamate, dodecane-1-sulfonate and (7,7)-dimethyl-2-oxo-bicyclo[2.2.1]-hept-1-yl-methanesulfonate;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**14.** A salt according to one or more of claims 1 to 13 selected from the group consisting of

[1] 1-[[5-(4-Chloro-phenyl)-1-(2,4-dichloro-phenyl)-4,5-dihydro-1H-pyrazole-3-carbonyl]-amino]-1-methyl-piperidinium iodide
[2] 1-[[5-(4-Chloro-phenyl)-1-(2,4-dichloro-phenyl)-4,5-dihydro-1H-pyrazole-3-carbonyl]-amino]-4-methyl-morpholin-4-ium iodide
[3] 1-[[5-(4-Chloro-phenyl)-1-(2,4-dichloro-phenyl)-4,5-dihydro-1H-pyrazo)e-3-carbonyl]-amino]-1-methyt-azepanium iodide
[4] 1-[[5-(4-Chloro-phenyl)-1-(2,4-dichloro-phenyl)-4,5-dihydro-1H-pyrazole-3-carbonyl]-amino]-2-methyl-octahydro-cyclopenta[c]pyrrolium iodide
[5] 1-[[5-(4-Chloro-phenyl)-1-(2,4-dichloro-phenyl)-4,5-dihydro-1H-pyrazole-3-carbonyl]-amino]-1-methyl-pyrrolidinium iodide
[6] (R)-1-[[5-(4-Chloro-phenyl)-1-(2,4-dichloro-phenyl)-4,5-dihydro-1H-pyrazole-3-carbonyl]-amino]-1-methyl-piperidinium iodide
[7] (S)-1-[[5-(4-Chloro-pheny))-1-(2,4-dichloro-phenyl)-4,5-dihydro-1H-pyrazole-3-carbonyl]-amino]-1-methyl-piperidinium iodide
[8] 1-[[5-(4-Chloro-phenyl)-1-(2,4-dichloro-phenyl)-4,5-dihydro-1H-pyrazole-3-carbonyl]-amino]-1-ethyl-piperidinium iodide
[9] (R)-1-[[5-(4-Chloro-phenyl)-1-(2,4-dichloro-phenyl)-4,5-dihydro-1H-pyrazole-3-carbonyl]-amino]-1-ethyl-piperidinium iodide
[10] (S)-1-[[5-(4-Chloro-phenyl)-1-(2,4-dichloro-phenyl)-4,5-dihydro-1H-pyrazole-3-carbonyl]-amino]-1-ethyl-piperidinium iodide
[11] 1-[[5-(4-Bromo-phenyl)-1-(2,4-dichloro-phenyl)-4,5-dihydro-1H-pyrazole-3-carbonyl]-amino]-1-methyl-piperidinium iodide
[12] 1-[[2,4-Dichloro-phenyl)-5-(4-iodo-phenyl)-4,5-dihydro-1H-pyrazole-3-carbonyl]-amino]-1-methyl-piperidinium iodide
[13]1-[[2,4-Dichloro-phenyl)-5-(4-fluoro-phenyl)-4,5-dihydro-1H-pyrazole-3-carbony)]-amino]-1-methy)-piperidinium iodide
[14] 1-[[2,4-Dichloro-phenyl)-5-(4-methoxy-phenyl)-4,5-dihydro-1H-pyrazole-3-carbonyl]-amino]-1-methyl-piperidinium iodide
[15] 1-[[2,4-Dichloro-phenyl)-5-(4-methoxy-phenyl)-4,5-dihydro-1H-pyrazole-3-carbonyl]-amino]-1-methyl-azepanium iodide
[16] 1-[[2,4-Dichloro-phenyl)-5-(4-fluoro-phenyl)-4,5-dihydro-1H-pyrazole-3-carbony)]-amino]-1-methyl-azepanium iodide
[17] 1-[[2,4-Dichloro-phenyl)-5-(4-iodo-phenyl)-4,5-dihydro-1H-pyrazole-3-carbonyl]-amino]-1-methyl-azepanium iodide
[18] 1-[[5-(4-Bromo-phenyl)-1-(2,4-dichloro-phenyl)-4,5-dihydro-1H-pyrazole-3-carbonyl]-amino]-1-methyl-azepanium iodide
[19] 1-[[5-(4-Bromo-phenyl)-1-(2,4-dichloro-phenyl)-4,5-dihydro-1H-pyrazole-3-carbonyl]-amino]-1-methyl-pyrrolidinium iodide
[20] 1-[[2,4-Dichloro-phenyl)-5-(4-fluoro-phenyl)-4,5-dihydro-1H-pyrazole-3-carbonyl]-amino]-1-methyl-pyrrolidinium iodide
[21] 1-[[2,4-Dichloro-phenyl)-5-(4-methoxy-phenyl)-4,5-dihydro-1H-pyrazole-3-carbonyl]-amino]-1-methyl-pyrrolidinium iodide
[22] cis-1-[[5-(4-Chloro-phenyl)-1-(2,4-dichloro-phenyl)-4-methyl-4,5-dihydro-1 H-pyrazole-3-carbonyl]-amino]-1-methyl-piperidinium iodide
[23] cis-1-[[5-(4-Chloro-phenyl)-1-(2,4-dichloro-phenyl)-4-ethyl-4,5-dihydro-1 H-pyrazole-3-carbonyl]-amino]-1-methyl-piperidinium iodide
[24] cis-1-[[5-(4-Bromo-phenyl)-1-(2,4-dichloro-phenyl)-4-methyl-4,5-dihydro-1 H-pyrazole-3-carbonyl]-amino]-1-methyl-piperidinium iodide
[25] cis-1-[[5-(4-Fluoro-phenyl)-1-(2,4-dichloro-phenyl)-4-ethyl-4,5-dihydro-1 H-pyrazole-3-carbonyl]-amino]-1-methyl-piperidinium iodide
[26] trans-1-[[5-(4-Chloro-phenyl)-1-(2,4-dichloro-phenyl)-4-methyl-4,5-dihydro-1H-pyrazole-3-carbonyl]-amino]-1-methyl-piperidinium iodide
[27] trans-1-[[5-(4-Chloro-phenyl)-1-(2,4-dichloro-phenyl)-4-ethyl-4,5-dihydro-1H-pyrazole-3-carbonyl]-amino]-1-methyl-piperidinium iodide
[28] trans-1-[[5-(4-Bromo-phenyl)-1-(2,4-dichloro-phenyl)-4-methyl-4,5-dihydro-1H-pyrazole-3-carbonyl]-amino]-1-methyl-piperidinium iodide
[29] trans-1-[(5-(4-Fluoro-phenyl)-1-(2,4-dichloro-phenyl)-4-ethyl-4,5-dihydro-1H-pyrazole-3-carbonyl]-amino]-1-methyl-piperidinium iodide
[30] cis-1-[[5-(4-Chloro-phenyl)-1-(2,4-dichloro-phenyl)-4-methyl-4,5-dihydro-1 H-pyrazole-3-carbonyl]-amino]-1-methyl-azepanium iodide
[31] cis-1-[[5-(4-Chloro-phenyl)-1-(2,4-dichloro-phenyl)-4-methyl-4,5-dihydro-1 H-pyrazole-3-carbonyl]-amino]-1-methyl-pyrrolidinium iodide
[32] trans-1-[[5-(4-Chloro-phenyl)-1-(2,4-dichloro-phenyl)-4-methyl-4,5-dihydro-1H-pyrazole-3-carbonyl]-amino]-1-methyl-pyrrolidinium iodide
[33] trans-1-[[5-(4-Chloro-phenyl)-1-(2,4-dichloro-phenyl)-4-methyl-4,5-dihydro-1H-pyrazole-3-carbonyl]-amino]-1-methyl-azepanium iodide
[34] cis-2-[[5-(4-Chloro-phenyl)-1-(2,4-dichloro-phenyl)-4-methyl-4,5-dihydro-1 H-pyrazole-3-carbonyl]-amino]-2-methyloctahydrocyclopenta[c]pyrrolium iodide
[35] trans-2-[[5-(4-Chloro-phenyl)-1-(2,4-dichloro-phenyl)-4-methyl-4,5-dihydro-1H-pyrazole-3-carbonyl]-amino]-2-methyloctahydrocyclopenta[c]pyrrolium iodide
[36] 2-[[5-(4-Chloro-phenyl)-1-(2,4-dichloro-phenyl)-4,4-dimethyl-4,5-dihydro-1H-pyrazole-3-carbonyl]-amino]-2-methyloctahydrocyclopenta[c]pyrrolium iodide
[37] 2-[[5-(4-Chloro-phenyl)-1-(2,4-dichloro-phenyl)-4,4-dimethyl-4,5-dihydro-1H-pyrazole-3-carbonyl]-amino]-1-methyl-piperidinium iodide
[38] cis-1-[(5-(4-Chloro-phenyl)-1-(2,4-dichloro-phenyl)-4-trifluormethyl-4,5-dihydro-1H-pyrazole-3-carbonyl]-amino]-1-methyl-piperidinium iodide
[39] cis-1-[[5-(4-Chloro-phenyl)-1-(2,4-dichloro-phenyl)-4-methoxy-4,5-dihydro-1H-pyrazole-3-carbonyl]-amino]-1-methyl-piperidinium iodide
[40] cis-1-[[5-(4-Chloro-phenyl)-1-(2,4-dichloro-phenyl)-4-cyano-4,5-dihydro-1 H-pyrazole-3-carbonyl]-amino]-1-methyl-piperidinium iodide
[41] 1-[[5-(5-Chloro-thiophen-2-yl)-1-(2,4-dichloro-phenyl)-4,5-dihydro-1H-pyrazole-3-carbonyl]-amino]-1-methyl-piperidinium iodide
[42] 1-[[5-(5-Bromo-thiophen-2-yl)-1-(2,4-dichloro-phenyl)-4,5-dihydro-1H-pyrazole-3-carbonyl]-amino]-1-methyl-piperidinium iodide
[43] 1-[[5-(4-Bromo-thiophen-2-yl)-1-(2,4-dichloro-phenyl)-4,5-dihydro-1H-pyrazole-3-carbonyl]-amino]-1-methyl-piperidinium iodide
[44] cis-1-[[5-(5-Chloro-thiophen-2-yl)-1-(2,4-dichloro-phenyl)-4-methyl-4,5-dihydro-1H-pyrazoie-3-carbonyl]-amino]-1-methyl-piperidinium iodide
[45] trans-1-[[5-(5-Chloro-thiophen-2-yl)-1-(2,4-dichloro-phenyl)-4-methyl-4,5-dihydro-1H-pyrazole-3-carbonyl]-amino]-1-methyl-piperidinium iodide
[46] cis-1-[[5-(5-Bromo-thiophen-2-yl)-1-(2,4-dichloro-phenyl)-4-methyl-4,5-dihydro-1H-pyrazole-3-carbonyl]-amino]-1-methyl-piperidinium iodide
[47] trans-1-[[5-(5-Bromo-thiophen-2-yl)-1-(2,4-dichloro-phenyl)-4-methyl-4,5-dihydro-1H-pyrazole-3-carbonyl]-amino]-1-methyl-piperidinium iodide
[48] cis-1-[[5-(4-Bromo-thiophen-2-yl)-1-(2,4-dichloro-phenyl)-4-methyl-4,5-dihydro-1H-pyrazole-3-carbonyl]-amino]-1-methyl-piperidinium iodide
[49] trans-1-[[5-(4-Bromo-thiophen-2-yl)-1-(2,4-dichloro-phenyl)-4-methyl-4,5-dihydro-1H-pyrazole-3-carbonyl]-amino]-1-methyl-piperidinium iodide
and
[50] cis-1-[[5-(4-Bromo-thiophen-2-yl)-1-(2,4-dichloro-phenyl)-4-hydroxy-4-methyl-4,5-dihydro-1H-pyrazole-3-carbonyl]-amino]-1-methyl-piperidinium iodide

optionally in form of one of its stereoisomers, preferably enantiomers or diastereomers, a racemate or in form of a mixture of at least two of its stereoisomers, preferably enantiomers and/or diastereomers, in any mixing ratio, or a corresponding solvate thereof.

**15.** Process for the preparation of a salt of general formula I according to one or more of claims 1 to 14, wherein $R^4$ represents hydrogen, **characterized in that** at least one compound of general formula $R^1$-C(=O)-H (general formula II), wherein $R^1$ has the meaning according to one or more of claims 1 to 14, is reacted with at least one compound of general formula III,

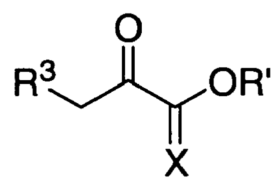

III,

wherein $R^3$ and X have the meaning according to one or more of claims 1 to 14 and R' represents a linear or branched $C_{1-6}$-alkyl radical, a potassium cation or a sodium cation, in a reaction medium, optionally in an inert atmosphere, optionally in the presence of at least one base, to yield at least one compound of general formula IV,

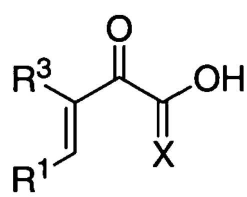

IV,

wherein $R^1$, X and $R^3$ have the meaning according to one or more of claims 1 to 14, which is optionally purified and/or isolated,
and at least one compound of general formula IV is reacted with at least one compound of general formula V,

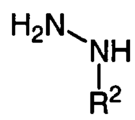

V,

or a corresponding salt thereof, wherein $R^2$ has the meaning according to one or more of claims 1 to 14, in a reaction medium, optionally in an inert atmosphere, optionally in the presence of at least one acid, to yield at least one compound of general formula VI,

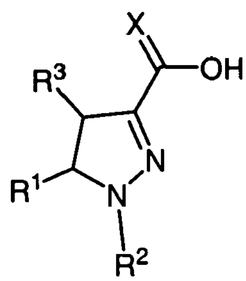

VI

wherein $R^1$, X, $R^2$ and $R^3$ have the meaning according to one or more of claims 1 to 14, which is optionally isolated and/or purified,
and at least one compound of general formula VI is reacted with an activating agent in a reaction medium, optionally in an inert atmosphere, to yield at least one compound of general formula VII,

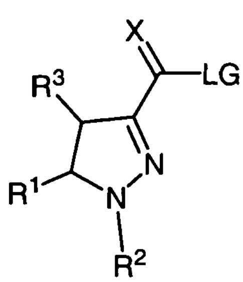

VII

wherein $R^1$, X, $R^2$ and $R^3$ have the meaning according to one or more of claims 1 to 14 and LG represents a leaving group, which is optionally purified and/or isolated,
and at least one compound of general formula VII is reacted with at least one compound of general formula $NH_2$-$NR^5R^6$, wherein $R^5$ and $R^6$ have the meaning according to one or more of claims 1 to 14, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula VIII,

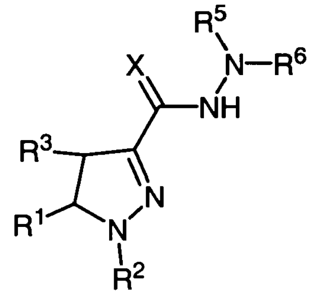

VIII

wherein $R^1$, $R^2$, $R^3$, X, $R^5$ and $R^6$ have the meaning according to one or more of claims 1 to 14, which is optionally purified and/or isolated;
or at least one compound of general formula VI is reacted with at least one compound of general formula $NH_2$-$NR^5R^6$, wherein $R^5$ and $R^6$ have the meaning according to one or more of claims 1 to 14, in a reaction medium, in the presence of at least one coupling agent, optionally in the presence of at least one base, to yield at least one compound of general VIII, which is optionally purified and/or isolated;
and at least one compound of general formula VIII is reacted with at least one compound of general formula A-B, wherein A and B have the meaning according to one or more of claims 1 to 14, in a reaction medium, to yield at least one salt of general formula I,

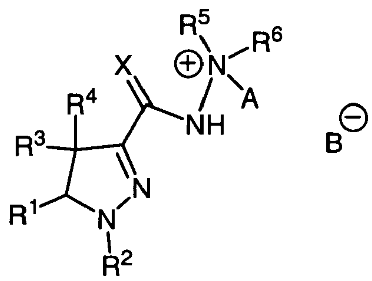

I

wherein A, B, $R^1$, $R^2$, $R^3$, X, $R^5$ and $R^6$ have the meaning according to one or more of claims 1 to 14 and $R^4$ represents hydrogen, which is optionally purified and/or isolated.

**16.** Medicament comprising at least one salt according to one or more of claims 1 to 14 and optionally at least one physiologically acceptable auxiliary agent.

**17.** Medicament according to claim 16 for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**18.** Medicament according to claim 16 or 17 for the prophylaxis and/or treatment of food intake disorders, preferably bulimia, anorexia, cachexia, obesity, type II diabetes mellitus (non-insuline dependent diabetes mellitus), more preferably obesity.

**19.** Medicament according to claim 16 or 17 for the prophylaxis and/or treatment of psychosis.

**20.** Medicament according to claim 16 or 17 for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**21.** Medicament according to claim 16 or 17 for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**22.** Medicament according to claim 16 or 17 for the prophylaxis and/or treatment of one or more disorders selected from the group consisting of bone disorders, preferably osteoporosis (e.g. osteoporosis associated with a genetic predisposition, sex hormone deficiency, or aging), cancer-associated bone disease or Paget's disease of bone; schizophrenia, anxiety, depression, epilepsy, neurodegenerative disorders, cerebella disorders, spinocerebella disorders, cognitive disorders, cranial trauma, head trauma, stroke panic attacks, peripheral neuropathy, glaucoma, migraine, Morbus Parkinson, Morbus Huntington, Morbus Alzheimer, Raynaud's disease, tremblement disorders, compulsive disorders, senile dementia, thymus disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**23.** Use of at least one salt according to one or more of claims 1 to 14 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**24.** Use of at least one salt according to one or more of claims 1 to 14 for the preparation of a medicament for the prophylaxis and/or treatment of food intake disorders, preferably bulimia, anorexia, cachexia, obesity, type II diabetes mellitus (non-insuline dependent diabetes mellitus), more preferably obesity.

**25.** Use of at least one salt according to one or more of claims 1 to 14 for the preparation of a medicament for the prophylaxis and/or treatment of psychosis.

**26.** Use of at least one salt according to one or more of claims 1 to 14 for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**27.** Use of at least one salt according to one or more of claims 1 to 14 for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**28.** Use of at least one salt according to one or more of claims 1 to 14 for the preparation of a medicament for the prophylaxis and/or treatment of one or more disorders selected from the group consisting of bone disorders, preferably osteoporosis (e.g. osteoporosis associated with a genetic predisposition, sex hormone deficiency, or aging), cancer-associated bone disease or Paget's disease of bone; schizophrenia, anxiety, depression, epilepsy, neurodegenerative disorders, cerebella disorders, spinocerebella disorders, cognitive disorders, cranial trauma, head trauma, stroke, panic attacks, peripheral neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

European Patent Office

# EUROPEAN SEARCH REPORT

Application Number
EP 05 38 4026

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 03/026647 A (SOLVAY PHARMACEUTICALS B.V; KRUSE, CORNELIS, G; LANGE, JOSEPHUS, H.M;) 3 April 2003 (2003-04-03)<br>* the whole document *<br>----- | 1-28 | C07D231/06<br>A61K31/4155<br>A61P25/00 |
| Y | WO 01/70700 A (SOLVAY PHARMACEUTICALS B.V) 27 September 2001 (2001-09-27)<br>* the whole document *<br>----- | 1-28 | |
| Y | SHIM J-Y ET AL: "Molecular interaction of the antagonist N-(piperidin-1-yl)-5-(4-chlorophenyl)-1-(2,4-dichlorophenyl)-4-methyl-1H-pyrazole-3-carboxamide with the CB1 cannabinoid receptor"<br>JOURNAL OF MEDICINAL CHEMISTRY, AMERICAN CHEMICAL SOCIETY. WASHINGTON, US,<br>vol. 45, no. 7, March 2002 (2002-03), pages 1447-1459, XP002968557<br>ISSN: 0022-2623<br>* figure 3 *<br>----- | 1-28 | TECHNICAL FIELDS SEARCHED (IPC) |
| Y | J. ADAMS; P. COWLEY: "Recent advances in the cannabinoids"<br>EXPERT OPIN. THER. PATENTS,<br>vol. 12, no. 10, 2002, pages 1475-1489, XP002353808<br>* page 1482 - page 1483 *<br>----- | 1-28 | C07D<br>A61K<br>A61P |
| E | WO 2005/077911 A (LABORATORIOS DEL DR. ESTEVE S.A; CUBERES ALTISEN, ROSA; FRIGOLA CONSTA) 25 August 2005 (2005-08-25)<br>* the whole document *<br>----- | 1-28 | |

The present search report has been drawn up for all claims

| Place of search | Date of completion of the search | Examiner |
|---|---|---|
| Munich | 14 November 2005 | Lauro, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 38 4026

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2005

| Patent document cited in search report | Publication date | Patent family member(s) | Publication date |
|---|---|---|---|
| WO 03026647 A | 03-04-2003 | BR 0212044 A | 17-08-2004 |
| | | CA 2456606 A1 | 03-04-2003 |
| | | CN 1555262 A | 15-12-2004 |
| | | HR 20040085 A2 | 31-08-2004 |
| | | HU 0401567 A2 | 28-01-2005 |
| | | JP 2005503427 T | 03-02-2005 |
| | | MX PA04002583 A | 18-06-2004 |
| | | NO 20041176 A | 21-06-2004 |
| | | US 2004248944 A1 | 09-12-2004 |
| | | ZA 200402099 A | 26-04-2005 |
| WO 0170700 A | 27-09-2001 | AU 4250101 A | 03-10-2001 |
| | | BR 0109457 A | 03-06-2003 |
| | | CA 2401832 A1 | 27-09-2001 |
| | | CN 1419546 A | 21-05-2003 |
| | | HK 1052349 A1 | 23-09-2005 |
| | | HU 0204519 A2 | 28-05-2003 |
| | | JP 2004500401 T | 08-01-2004 |
| | | NO 20024531 A | 19-11-2002 |
| | | PL 358101 A1 | 09-08-2004 |
| | | SK 13522002 A3 | 04-03-2003 |
| | | US 2001053788 A1 | 20-12-2001 |
| WO 2005077911 A | 25-08-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992
- *Synthetic Communications,* 1996, vol. 26 (11), 2229-33
- *Synlett,* 2001, 147-149
- *Tetrahedron Lett.,* 2004, vol. 45, 4977
- *Tetrahedron Lett.,* 1998, vol. 39, 1487
- *Tetrahedron Lett.,* 2002, vol. 43, 7685
- Protective groups in Organic Chemistry. Plenum Press, 1973
- **T.W. GREENE ; P.G.M. WUTS.** Protective Groups in Organic Chemistry. John Wiley & sons, 1991
- **R. B. SILVERMAN.** The Organic Chemistry of Drug Design and Drug Action. Academic Press, 1992
- Pharmaceutics: The Science of Dosage Forms. Churchill Livingstone, 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRD Press Inc, 1983, vol. I
- Oral Drug Delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encyclopedia of Controlled Drug Delivery. John Wiley & Sons, Inc, 1999, vol. 2, 698-728
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol.,* 2003, vol. 138 (4), 544-553